# EUROPEAN PATENT APPLICATION

(11) **EP 4 589 008 A1**
(43) Date of publication of application: **23.07.2025**
(21) Application number: 23865507.0
(22) Date of filing: 12.09.2023
(51) Int. Cl.: C12N 15/63, C07K 19/00, C12N 1/15, C12N 1/19, C12N 1/21, C12N 5/10, C12N 9/12, C12N 9/16, C12N 15/10, C12N 15/11, C12N 15/54, C12N 15/55, C12N 15/62

(54) **DOUBLE STRANDED CIRCULAR DNA VECTOR, METHOD FOR PRODUCING LINEAR COVALENTLY CLOSED DNA, AND FUSION POLYPEPTIDE CONTAINING PROTELOMERASE AND ENDONUCLEASE**

(30) Priority: 12.09.2022 JP 2022144282; 12.09.2022 JP 2022144285
(71) Applicant: Kaneka Corporation, Osaka-shi, Osaka 530-8288 (JP)
(72) Inventor: SHIGETA, Mitsuki, Takasago-shi, Hyogo 676-8688 (JP); OSHIMA, Kanji, Takasago-shi, Hyogo 676-8688 (JP); NISHI, Teruyuki, Takasago-shi, Hyogo 676-8688 (JP); MAEDA, Hirofumi, Takasago-shi, Hyogo 676-8688 (JP); MINAKUCHI, Kazunobu, Takasago-shi, Hyogo 676-8688 (JP)
(74) Representative: Hoffmann Eitle
(86) International application number: PCT/JP2023/033127
(87) International publication number: WO 2024/058155

(57) **Abstract**

A problem to be addressed by the present invention is to provide a new method of producing a linear covalently closed DNA easily and efficiently. Provided is a double-stranded circular DNA vector, comprising a protelomerase gene sequence encoding a protelomerase or an active fragment thereof, an endonuclease gene sequence encoding an endonuclease or an active fragment thereof, a pair of protelomerase recognition sequences which are recognized by said protelomerase or an active fragment thereof for cleaving said vector, at least one endonuclease recognition sequence which is recognized by said endonuclease or an active fragment thereof for cleaving said vector, and a nucleic acid sequence of interest, wherein said protelomerase gene sequence, said endonuclease gene sequence, and said endonuclease recognition sequence are placed in the same region between said pair of protelomerase recognition sequences, and said nucleic acid sequence of interest is placed in another region between said pair of protelomerase recognition sequences in said double-stranded circular DNA vector, and wherein said protelomerase gene sequence and said endonuclease gene sequence are placed under the control of a promoter which is capable of regulating the expression.

## Description

### Technical Field

The present invention relates to a double-stranded circular DNA vector, a transformed cell, and a method of producing a linear covalently closed DNA.

### Background Art

A double-stranded circular DNA vector is widely utilized as a DNA vector which can be readily manipulated. A double-stranded circular DNA vector comprises a gene sequence of interest, and in addition, comprises, e.g., a replication origin sequence which is a sequence necessary for maintenance and replication of a plasmid, and an antibiotic resistance gene. When a double-stranded circular DNA vector is transduced to a tissue or cell of an animal, these sequences are also delivered together with the gene sequence of interest, which are undesirable from the viewpoint of avoiding immunogenicity.

A linear covalently closed DNA (LCC DNA) is used as means for removing an unnecessary sequence from a double-stranded circular DNA vector to obtain only a gene sequence of interest (Patent Literature 1 and Patent Literature 2).

A linear covalently closed DNA is a linear double-stranded DNA in which the terminal parts are closed in a hairpin structure, and can be produced by utilizing the activity whereby a double-stranded DNA cleaved by a protelomerase forms a hairpin structure at the cleaved ends. For example, a double-stranded circular DNA vector in which a gene sequence of interest is placed between a pair of protelomerase recognition sequences is provided. Next, the protelomerase activity is allowed to act on this double-stranded circular DNA vector, thus making it possible to produce a linear covalently closed DNA comprising only a gene sequence of interest and having ends closed in a hairpin structure. A linear covalently closed DNA produced in this manner has resistance to an exonuclease, and accordingly, is stable and small in size, thus having the advantage of high efficiency of transduction to a cell. In addition, placing a protelomerase recognition sequence allows removal of a replication origin sequence, an antibiotic resistance gene, and the like from a linear covalently closed DNA comprising a gene of interest, thus making it possible to reduce a risk such as immunogenicity.

Patent Literature 1 discloses a method of producing a linear covalently closed DNA in vitro. The method described in Patent Literature 1 is a method in which a double-stranded DNA molecule is synthesized in vitro using a DNA polymerase, and then, the double-stranded DNA molecule is cleaved through treatment with a protelomerase to produce a linear covalently closed DNA. In this method, however, the procedure for causing various enzymes to react in vitro is complicated. Furthermore, simultaneously with a first linear covalently closed DNA comprising a gene of interest, an unnecessary second linear covalently closed DNA derived from the vector sequence other than the gene of interest is generated, and needs to be degraded and removed, thus necessitating various isolated enzymes, and accordingly involving high cost.

Patent Literature 2 discloses a method of producing a linear covalently closed DNA in a cell. The method described in Patent Literature 2 is a method in which a double-stranded DNA molecule is amplified in a cell, and then, the expression of a protelomerase is induced in a cell to cleave the double-stranded DNA molecule, thus producing a linear covalently closed DNA. However, simultaneously with a first linear covalently closed DNA comprising a gene of interest, an unnecessary second linear covalently closed DNA derived from the vector sequence other than the gene of interest is generated also in this method. Because of this, there is still a demand for a procedure whereby the second linear covalently closed DNA is selectively degraded and removed in vitro after the first and second linear covalently closed DNAs are isolated from *Escherichia coli.*

Accordingly, there is a demand for a new method of producing a linear covalently closed DNA more easily and more efficiently.

### Citation List

### Patent Literature

Patent Literature 1: US9109250B2
Patent Literature 2: WO2022/209987A1

### Summary of Invention

### Technical Problem

An object of the present invention is to provide a new method of producing a linear covalently closed DNA easily and efficiently.

### Solution to Problem

In a reaction which allows a double-stranded circular DNA vector to be cleaved with a protelomerase activity to generate a linear covalently closed DNA, a first linear covalently closed DNA comprising a gene of interest is generated, and in addition, a linear covalently closed DNA derived from the vector sequence other than the gene of interest is generated as an unnecessary second linear covalently closed DNA.

To solve the above-described problem, the present inventors have conceived a method in which all elements necessary to generate a linear covalently closed DNA in a cell are transduced to one double-stranded circular DNA vector to easily produce a linear covalently closed DNA. Specifically, to generate a first linear covalently closed DNA from a double-stranded circular DNA vector in a cell, and induce degradation of an unnecessary second linear covalently closed DNA that is generated simultaneously, the followings are placed in one double-stranded circular DNA vector: a gene of interest located between protelomerase recognition sequences; and a fusion gene sequence encoding a fusion polypeptide comprising a protelomerase and an endonuclease, and an endonuclease recognition sequence, located in another region between the protelomerase recognition sequences. The endonuclease and the endonuclease recognition sequence are introduced for inducing degradation of the unnecessary second linear covalently closed DNA.

The present inventors have introduced the above-described double-stranded circular DNA vector to *Escherichia coli* as a host cell, induced the expression of a fusion polypeptide comprising a protelomerase and an endonuclease, and consequently found that inducing degradation of a second linear covalently closed DNA enables a first linear covalently closed DNA comprising a gene sequence of interest to be produced easily and efficiently.

The present invention is advantageous in that all elements necessary to generate a linear covalently closed DNA in a cell are introduced in one double-stranded circular DNA vector, thus enabling a linear covalently closed DNA to be produced very easily. The present invention is based on the above-described findings, and provides the following.

(1) A double-stranded circular DNA vector, comprising:
   a fusion gene sequence encoding a fusion polypeptide comprising a protelomerase or an active fragment thereof and an endonuclease or an active fragment thereof,
   a pair of protelomerase recognition sequences which are recognized by said protelomerase or an active fragment thereof for cleaving said vector,
   at least one endonuclease recognition sequence which is recognized by said endonuclease or an active fragment thereof for cleaving said vector, and
   a gene sequence of interest encoding a protein of interest or a fragment thereof,
   wherein said fusion gene sequence and said endonuclease recognition sequence are placed in the same region between said pair of protelomerase recognition sequences, and said gene sequence of interest is placed in another region between said pair of protelomerase recognition sequences in said double-stranded circular DNA vector, and
   wherein said fusion gene sequence is placed under the control of a promoter which is capable of regulating the expression.
(2) The double-stranded circular DNA vector of (1), wherein said protelomerase or an active fragment thereof is placed at an N-terminal side of said endonuclease or an active fragment thereof in said fusion polypeptide.
(3) The double-stranded circular DNA vector of (1) or (2), wherein said fusion polypeptide further comprises a linker sequence between said protelomerase or an active fragment thereof and said endonuclease or an active fragment thereof.
(4) The double-stranded circular DNA vector of any one of (1) to (3), further comprising a spacer sequence which is placed between said endonuclease recognition sequence and said protelomerase recognition sequence.
(5) The double-stranded circular DNA vector of (4), wherein said spacer sequence is 200 bases in length or less.
(6) The double-stranded circular DNA vector of any one of (1) to (3), wherein said endonuclease recognition sequence is placed adjacent to one of said protelomerase recognition sequences.
(7) The double-stranded circular DNA vector of any one of (1) to (6), which is a plasmid.
(8) The double-stranded circular DNA vector of any one of (1) to (7), wherein said protelomerase is TelN protelomerase, TelA protelomerase, or TelK protelomerase.
(9) The double-stranded circular DNA vector of any one of (1) to (8), wherein said endonuclease is a homing endonuclease.
(10) A transformed cell comprising the double-stranded circular DNA vector of any one of (1) to (9).
(11) A method of producing a linear covalently closed DNA, comprising
   a culture step of culturing the transformed cell of (10),
   an expression-induction step of inducing the expression of said fusion polypeptide in the transformed cell after said culture step, and
   a DNA extraction step of extracting DNA from the transformed cell after said expression-induction step.
(12) The method of (11), further comprising a separation step of separating the linear covalently closed DNA after said DNA extraction step.
(13) The method of (11) or (12), further comprising a transduction step of transducing the double-stranded circular DNA vector of any one of (1) to (9) to a host cell before said culture step for producing the transformed cell.
(14) The method of any one of (11) to (13), wherein said host cell or said transformed cell expresses an exonuclease.
(15) A fusion polypeptide comprising a protelomerase or an active fragment thereof at an N-terminal side and an endonuclease or an active fragment thereof at a C-terminal side, or a nucleic acid encoding the same.

In addition, to generate a first linear covalently closed DNA from a double-stranded circular DNA vector in a cell, and induce degradation of an unnecessary second linear covalently closed DNA that is generated simultaneously, the followings are placed in one double-stranded circular DNA vector: a gene of interest located between protelomerase recognition sequences; and a protelomerase gene sequence, an endonuclease gene sequence, and an endonuclease recognition sequence, located in another region between the protelomerase recognition sequences. The endonuclease and the endonuclease recognition sequence are introduced for inducing degradation of the unnecessary second linear covalently closed DNA.

The present inventors have transduced the above-described double-stranded circular DNA vector to *Escherichia coli* as a host cell, induced the expression of a protelomerase and an endonuclease, and consequently found that inducing degradation of a second linear covalently closed DNA enables a first linear covalently closed DNA comprising a gene sequence of interest to be produced easily and efficiently. The present invention is based on the above-described findings, and provides the following.

(1) A double-stranded circular DNA vector, comprising
   a protelomerase gene sequence encoding a protelomerase or an active fragment thereof,
   an endonuclease gene sequence encoding an endonuclease or an active fragment thereof,
   a pair of protelomerase recognition sequences which are recognized by said protelomerase or an active fragment thereof for cleaving said vector,
   at least one endonuclease recognition sequence which is recognized by said endonuclease or an active fragment thereof for cleaving said vector, and
   a nucleic acid sequence of interest (for example, a gene sequence of interest encoding a protein of interest or a fragment thereof),
   wherein said protelomerase gene sequence, said endonuclease gene sequence, and said endonuclease recognition sequence are placed in the same region between said pair of protelomerase recognition sequences, and said nucleic acid sequence of interest (for example, said gene sequence of interest) is placed in another region between said pair of protelomerase recognition sequences in said double-stranded circular DNA vector, and
   wherein said protelomerase gene sequence and said endonuclease gene sequence are placed under the control of a promoter which is capable of regulating the expression.
(2) The double-stranded circular DNA vector of (1), which is a plasmid.
(3) The double-stranded circular DNA vector of (1) or (2), wherein said protelomerase is TelN protelomerase, TelA protelomerase, or TelK protelomerase.
(4) The double-stranded circular DNA vector of any one of (1) to (3), wherein said endonuclease is a homing endonuclease.
(5) The double-stranded circular DNA vector of (4), wherein said homing endonuclease is I-SceI homing endonuclease, I-CeuI homing endonuclease, or I-CreI homing endonuclease.
(6) A transformed cell comprising the double-stranded circular DNA vector of any one of (1) to (5).
(7) The transformed cell of (6), wherein the cell is a bacterial cell or a eukaryotic cell.
(8) The transformed cell of (7), wherein the bacterial cell is *Escherichia coli* or *Bacillus subtilis.*
(9) The transformed cell of any one of (6) to (8), which expresses an exonuclease.
(10) A method of producing a linear covalently closed DNA, comprising
   a culture step of culturing the transformed cell of any one of (6) to (9),
   an expression-induction step of inducing the expression of said protelomerase or an active fragment thereof and said endonuclease or an active fragment thereof in the transformed cell after said culture step, and
   a DNA extraction step of extracting DNA from the transformed cell after said expression-induction step.
(11) The method of (10), further comprising a separation step of separating the linear covalently closed DNA after said DNA extraction step.
(12) The method of (10) or (11), further comprising a transduction step of transducing the double-stranded circular DNA vector of any one of (1) to (5) to a host cell before said culture step for producing the transformed cell.
(13) The method of any one of (10) to (12), wherein said transformed cell expresses an exonuclease.

The present invention further provides the following.
(1) A double-stranded circular DNA vector, comprising
   a protelomerase gene sequence encoding a protelomerase or an active fragment thereof,
   an endonuclease gene sequence encoding an endonuclease or an active fragment thereof,
   a pair of protelomerase recognition sequences which are recognized by said protelomerase or an active fragment thereof for cleaving said vector,
   at least one endonuclease recognition sequence which is recognized by said endonuclease or an active fragment thereof for cleaving said vector, and
   a nucleic acid sequence of interest (for example, a gene sequence of interest encoding a protein of interest or a fragment thereof),
   wherein said protelomerase gene sequence, said endonuclease gene sequence, and said endonuclease recognition sequence are placed in the same region between said pair of protelomerase recognition sequences, and said nucleic acid sequence of interest (for example, said gene sequence of interest) is placed in another region between said pair of protelomerase recognition sequences in said double-stranded circular DNA vector, and
   wherein said protelomerase gene sequence and said endonuclease gene sequence are placed under the control of a promoter which is capable of regulating the expression.
(2) The double-stranded circular DNA vector of (1), wherein said protelomerase gene sequence and said endonuclease gene sequence encode a fusion polypeptide comprising said protelomerase or an active fragment thereof and said endonuclease or an active fragment thereof.
(3) The double-stranded circular DNA vector of (2), wherein said protelomerase or an active fragment thereof is placed at an N-terminal side of said endonuclease or an active fragment thereof in said fusion polypeptide.
(4) The double-stranded circular DNA vector of (2) or (3), wherein said fusion polypeptide further comprises a linker sequence between said protelomerase or an active fragment thereof and said endonuclease or an active fragment thereof.
(5) The double-stranded circular DNA vector of any one of (1) to (4), further comprising a spacer sequence which is placed between said endonuclease recognition sequence and said protelomerase recognition sequence.
(6) The double-stranded circular DNA vector of (5), wherein said spacer sequence is 200 bases in length or less.
(7) The double-stranded circular DNA vector of any one of (1) to (6), wherein said endonuclease recognition sequence is placed adjacent to one of said protelomerase recognition sequences.
(8) The double-stranded circular DNA vector of any one of (1) to (7), which is a plasmid.
(9) The double-stranded circular DNA vector of any one of (1) to (8), wherein said protelomerase is TelN protelomerase, TelA protelomerase, or TelK protelomerase.
(10) The double-stranded circular DNA vector of any one of (1) to (9), wherein said endonuclease is a homing endonuclease.
(11) The double-stranded circular DNA vector of (10), wherein said homing endonuclease is I-SceI homing endonuclease, I-CeuI homing endonuclease, or I-CreI homing endonuclease.
(12) A transformed cell comprising the double-stranded circular DNA vector of any one of (1) to (11).
(13) The transformed cell of (12), which is a bacterial cell or a eukaryotic cell.
(14) The transformed cell of (13), wherein the bacterial cell is *Escherichia coli* or *Bacillus subtilis.*
(15) The transformed cell of any one of (12) to (14), which expresses an exonuclease.
(16) A method of producing a linear covalently closed DNA, comprising
   a culture step of culturing the transformed cell of any one of (12) to (15),
   an expression-induction step of inducing the expression of said protelomerase or an active fragment thereof and said endonuclease or an active fragment thereof in the transformed cell after said culture step, and
   a DNA extraction step of extracting DNA from the transformed cell after said expression-induction step.
(17) The method of (16), further comprising a separation step of separating the linear covalently closed DNA after said DNA extraction step.
(18) The method of (16) or (17), further comprising a transduction step of transducing the double-stranded circular DNA vector of claim 1 to a host cell before said culture step for producing the transformed cell.
(19) The method of any one of (16) to (18), wherein said transformed cell expresses an exonuclease.
(20) A fusion polypeptide comprising a protelomerase or an active fragment thereof at an N-terminal side and an endonuclease or an active fragment thereof at a C-terminal side, or a nucleic acid encoding the same.

The present specification encompasses the disclosure of Japanese Patent Application No. 2022-144282 and Japanese Patent Application No. 2022-144285 that serve as the basis of the priority of the present application.

### Advantageous Effects of Invention

The present invention provides a new method of producing a linear covalently closed DNA easily and efficiently.

### Brief Description of Drawings

[Figure 1] Figure 1 illustrates a control vector for producing a linear covalently closed DNA.
[Figure 2] Figure 2 illustrates a separate expression vector for producing a linear covalently closed DNA.
[Figure 3] Figure 3 illustrates a fusion polypeptide in which fusion polypeptide I-SceI homing endonuclease is fused at a C-terminal side of TelN protelomerase via a linker sequence, and DNA.
[Figure 4] Figure 4 illustrates a fusion expression vector for producing a linear covalently closed DNA.
[Figure 5] Figure 5 illustrates the electrophoresis results of a linear covalently closed DNA. A linear covalently closed DNA comprising a nucleic acid encoding a packaging protein is illustrated as a "linear covalently closed DNA of interest", and a linear covalently closed DNA comprising nucleic acids encoding TelN protelomerase and I-SceI homing endonuclease is illustrated as an "unnecessary linear covalently closed DNA". "Con" represents a control vector, and "M" represents a DNA marker (N3200, manufactured by New England Biolabs).
[Figure 6] Figure 6 illustrates the results of a study on expression-inducing conditions. In the drawing, "Method A" shows a method in which shaking culture was performed at 37°C for 6 hours, and "Method B" shows a method in which shaking culture was performed at 30°C for 4 hours, and then, shaking culture was performed at 37°C for 2 hours. pH represents the pH of a Plusgrow II medium added for expression induction. "M" represents a DNA marker (N3200, manufactured by New England Biolabs).
[Figure 7] Figure 7 illustrates a control vector (T6) for producing a linear covalently closed DNA, as one of the two vectors produced in Comparative Example 1.
[Figure 8] Figure 8 illustrates an I-SceI vector for producing a linear covalently closed DNA, as one of the two vectors produced in Comparative Example 1.
[Figure 9] Figure 9 illustrates the electrophoresis results of a linear covalently closed DNA in Comparative Example 2. A linear covalently closed DNA comprising a nucleic acid encoding a packaging protein is illustrated as a "linear covalently closed DNA of interest", and the other linear covalently closed DNA is illustrated as an "unnecessary linear covalently closed DNA". In the drawing, a band having a high degree of electrophoresis corresponds to a remaining I-SceI vector. "M" represents a DNA marker (N3200, manufactured by New England Biolabs).

### Description of Embodiments

### 1. Double-stranded circular DNA vector

### 1-1. Overview

A first aspect of the present invention is a double-stranded circular DNA vector. A double-stranded circular DNA vector of the present aspect comprises, a fusion gene sequence encoding a fusion polypeptide comprising a protelomerase or an active fragment thereof and an endonuclease or an active fragment thereof, and an endonuclease recognition sequence in the same region between a pair of protelomerase recognition sequences; and comprises a nucleic acid sequence of interest in another region between the pair of protelomerase recognition sequences.

The double-stranded circular DNA vector of the present aspect makes it possible to easily and efficiently produce a linear covalently closed DNA comprising a nucleic acid sequence of interest.

### 1-2. Definitions of terms

The following terms frequently used herein will be defined.

As used herein, a "linear covalently closed DNA (LCC DNA)" refers to a linear double-stranded DNA the ends of which are closed in a hairpin structure. At each end of a linear covalently closed DNA, the 5' end and the 3' end of two DNA strands are linked to form a hairpin structure. In the linear covalently closed DNA, the terminal parts protected by the hairpin structure have resistance to degradation by an exonuclease.

As used herein, a "protelomerase" means a polypeptide having an activity of recognizing a specific sequence in a double-stranded DNA molecule, cleaving the double-stranded DNA molecule at a position within or in the vicinity of the recognized sequence, and linking the 5' end and the 3' end at the cleaved ends to form a covalently closed end (the activity is hereinafter referred to as a "protelomerase activity"). Herein, the kind of the protelomerase is not particularly limited, as long as having the above activity. Examples include *Agrobacterium fabrum-*derived protelomerase (TelA protelomerase, ACCESSION No. AAK88254), *Halomonas virus HAP1*-derived protelomerase (ACCESSION No. ABY90402), *Vibrio virus* VP882-derived protelomerase (ACCESSION No. ABM73418), *Klebsiella phage phiKO2-*derived protelomerase (TelK protelomerase, ACCESSION No. AAR83042), *Rhizobium pusense-*derived protelomerase (ACCESSION No. QKJ91773), *Feldmannia species virus-*derived protelomerase (ACCESSION No. ACH46812), *Vibrio phage vB_VpaM_MAR*-derived protelomerase (ACCESSION No. AFV81380), *Yersinia phage PY54*-derived protelomerase (Tel protelomerase, ACCESSION No. CAD91792), *Escherichia* virus N15-derived protelomerase (TelN protelomerase, ACCESSION No. AAB81106), and variants of any of the above protelomerases. In this regard, a protelomerase herein is not comprised in the below-described endonuclease.

As used herein, the "active fragment" of a protelomerase is a polypeptide fragment comprising a partial region of a protelomerase, and having a protelomerase activity, for example, having 1% or more, 10% or more, 20% or more, 30% or more, 40% or more, 50% or more, 60% or more, 70% or more, 80% or more, 90% or more, 95% or more, 98% or more, or the equivalent or more of the activity of the full-length protein. The length of the amino acids of the polypeptide constituting the active fragment is not particular limited. For example, the fragment of a protelomerase may be a region of at least 100, 150, 200, 250, 300, 350, or 400 consecutive amino acids comprising a catalytic domain.

As used herein, a "protelomerase gene" is a gene encoding a protelomerase or an active fragment thereof. Examples of the protelomerase gene include a gene encoding any of the above-described protelomerases or an active fragment thereof. Examples include a protelomerase gene encoding the TelN protelomerase consisting of the amino acid sequence of SEQ ID NO: 24, for example, the TelN protelomerase gene consisting of the base sequence of SEQ ID NO: 2.

As used herein, a "protelomerase recognition sequence" means a sequence which is recognized by a protelomerase. A protelomerase recognizes a protelomerase recognition sequence, cleaves a double-stranded DNA molecule at a position within or near the protelomerase recognition sequence, and recombines the cleaved ends. It is known that a protelomerase recognition sequence is a palindrome sequence or a palindrome-like sequence.

As used herein, an "endonuclease" means an enzyme having an activity of cleaving a phosphodiester bond in the interior side of a nucleotide strand. Among endonucleases, an endonuclease which recognizes a specific sequence, and cleaves a double-stranded DNA molecule within or near the sequence is particularly referred to as a restriction enzyme. Examples of the restriction enzyme include naturally-occurring restriction enzymes and besides, artificial restriction enzymes such as TALEN and zinc finger nuclease (ZFN), genome-editing enzymes such as a Cas9 protein, and a variant of any of the endonucleases. In this regard, the endonuclease may be any of an enzyme having an activity of cleaving two DNA strands and an enzyme (Nickase) which cleaves only one of the DNA strands.

Among the restriction enzymes, a restriction enzyme found in an intein, intron, or the like is particularly referred to as a "homing endonuclease". It is generally known that a homing endonuclease recognizes a long recognition sequence of 12 to 40 bp. It is also known that the recognition sequence is not a palindrome sequence. Herein, the kind of the homing endonuclease is not particularly limited. Examples include I-SceI (ACCESSION No. NP009324), I-CeuI (ACCESSION No. CAA78934), I-CreI (ACCESSION No. CAA26008), I-AniI, I-ChuI, I-DmoI, I-CsmI, PI-SceI, PI-TIiI, PI-MtuI, I-SceII, I-SceIII, HO, PI-CivI, PI-CtrI, PI-AaeI, PI-BsuI, PI-DhaI, PI-DraI, PI-MavI, PI-MchI, PI-MfuI, PI-MflI, PI-MgaI, PI-MgoI, PI-MinI, PI-MkaI, PI-MmaI, PI-MshI, PI-MsmI, I-MsoI, PI-MthI, PI-MtuI, PI-MxeI, PI-NpuI, PI-PfuI, PI-RmaI, PI-SpbI, PI-SspI, PI-FacI, PI-MjaI, PI-PhoI, PI-TagI, PI-ThyI, PI-TkoI, PI-TspI, and a variant of any of the homing endonucleases. Among these, I-SceI, I-ChuI, I-DmoI, I-CreI, I-CsmI, PI-PfuI, PI-SceI, PI-TliI, I-MsoI, PI-MtuI, I-CeuI, I-SceII, I-SceIII, HO, a variant of any of these homing endonucleases, and the like are preferable. I-SceI, I-ChuI, I-DmoI, I-CreI, I-CsmI, PI-SceI, PI-PfuI, PI-TliI, I-MsoI, PI-MtuI, I-CeuI, a variant of any of these homing endonucleases, and the like are more preferable. I-SceI, I-CeuI, I-CreI, a variant of any of these homing endonucleases, and the like are still more preferable.

As used herein, the "active fragment" of an endonuclease is a polypeptide fragment comprising a partial region of an endonuclease, and having an endonuclease activity, for example, having 1% or more, 10% or more, 20% or more, 30% or more, 40% or more, 50% or more, 60% or more, 70% or more, 80% or more, 90% or more, 95% or more, 98% or more, or the equivalent or more of the activity of the full-length protein. The length of the amino acids of the polypeptide constituting the active fragment is not particular limited. For example, the fragment in an endonuclease may be a region of at least 100, 150, 200, 250, 300, 350, or 400 consecutive amino acids comprising a catalytic domain.

As used herein, an "endonuclease gene" is a gene encoding an endonuclease or an active fragment thereof. Examples of the endonuclease gene include a gene encoding any of the above-described endonucleases or an active fragment thereof. Examples include a gene encoding I-SceI homing endonuclease consisting of the amino acid sequence of SEQ ID NO: 25, for example, an I-SceI homing endonuclease gene consisting of the base sequence of SEQ ID NO: 4.

As used herein, an "endonuclease recognition sequence" means a sequence which is recognized by an endonuclease. An endonuclease recognizes an endonuclease recognition sequence, and cleaves a double-stranded DNA molecule at a position within or near the endonuclease recognition sequence.

In the present invention, a "double-stranded circular DNA vector" means a circular double-stranded DNA molecule retainable and/or replicable in a cell. A double-stranded circular DNA vector can comprise: a sequence necessary for intracellular maintenance and replication, for example, an origin of replication; a gene encoding an antibiotic-resistant protein; and/or the like. The double-stranded circular DNA vector may be, for example, a plasmid vector or a bacmid vector. In addition, the vector may be a shuttle vector replicable in a bacterium such as *Escherichia coli* and a mammal cell.

### 1-3. Configuration

A double-stranded circular DNA vector of the present aspect comprises a fusion gene sequence, a pair of protelomerase recognition sequences, at least one endonuclease recognition sequence, and a nucleic acid sequence of interest. In the double-stranded circular DNA vector of the present aspect, the fusion gene sequence and the endonuclease recognition sequence are placed within the same region between the pair of protelomerase recognition sequences, and the nucleic acid sequence of interest is placed within another region between the pair of protelomerase recognition sequences. In addition, the fusion gene sequence is placed under the control of a promoter which is capable of regulating the expression.

As used herein, a "fusion gene sequence" means a gene sequence encoding a fusion polypeptide containing a protelomerase or an active fragment thereof and an endonuclease or an active fragment thereof. The fusion gene sequence may undergo codon optimization in accordance with the codon usage in a cell to which a double-stranded circular DNA vector is transduced.

A "fusion polypeptide comprising a protelomerase or an active fragment thereof and an endonuclease or an active fragment thereof" may be any of the following: a fusion polypeptide in which a protelomerase or an active fragment thereof is placed at an N-terminal side of an endonuclease or an active fragment thereof; and a fusion polypeptide in which a protelomerase or an active fragment thereof is placed at a C-terminal side of an endonuclease or an active fragment thereof. Preferably, the protelomerase or an active fragment thereof is placed at an N-terminal side of the endonuclease or an active fragment thereof.

The protelomerase or an active fragment thereof comprised in the fusion polypeptide is not particularly limited to any kind, as long as having a protelomerase activity, and may be those which do not recognize a sequence other than the pair of protelomerase recognition sequences in a double-stranded circular DNA vector, and particularly do not recognize a nucleic acid sequence of interest. In addition, the genomic sequence of a cell to which a double-stranded circular DNA vector is transduced may be those which do not have the protelomerase recognition sequence. For example, the length of the protelomerase recognition sequence which is recognized by the protelomerase or an active fragment thereof comprised in the fusion polypeptide may be, but is not limited to, 5 bp or more, 6 bp or more, 7 bp or more, 8 bp or more, 9 bp or more, 10 bp or more, 15 bp or more, 20 bp or more, 25 bp or more, 30 bp or more, 35 bp or more, 40 bp or more, 45 bp or more, 50 bp or more, 55 bp or more, 60 bp or more, 65 bp or more, 70 bp or more, 80 bp or more, or 90 bp or more. Particularly preferable examples of the protelomerases include TelN protelomerase, TelA protelomerase, TelK protelomerase, and a variant thereof. The variant is not particularly limited, and may be suitably selected in accordance with the purpose. For example, the variant may have a homology of 80% or more, 90% or more, 95% or more, or 99% or more with the original amino acid sequence.

The endonuclease or an active fragment thereof comprised in the fusion polypeptide is not particularly limited, as long as having an endonuclease activity, and may be those which do not recognize a sequence other than the endonuclease recognition sequence in a double-stranded circular DNA vector, and particularly do not recognize a nucleic acid sequence of interest. In addition, the genomic sequence of a cell to which a double-stranded circular DNA vector is transduced may be those which do not have the endonuclease recognition sequence. The endonuclease or an active fragment thereof may be a restriction enzyme, or may be Nickase. For example, the length of the endonuclease recognition sequence which is recognized by the endonuclease or an active fragment thereof comprised in the fusion polypeptide may be, but is not limited to, 4 bp or more, 5 bp or more, 6 bp or more, 7 bp or more, 8 bp or more, 9 bp or more, 10 bp or more, 15 bp or more, 20 bp or more, 25 bp or more, 30 bp or more, 35 bp or more, 40 bp or more, 45 bp or more, 50 bp or more, 60 bp or more, 70 bp or more, 80 bp or more, 90 bp or more, or 100 bp or more.

In one embodiment of the present invention, the endonuclease may be a homing endonuclease. Examples of the homing endonuclease include, but are not limited to, I-SceI, I-CeuI, I-CreI, I-AniI, I-ChuI, I-DmoI, I-CsmI, PI-SceI, PI-TIiI, PI-MtuI, I-CeuI, I-SceII, I-SceIII, HO, PI-CivI, PI-CtrI, PI-AaeI, PI-BsuI, PI-DhaI, PI-DraI, PI-MavI, PI-MchI, PI-MfuI, PI-MflI, PI-MgaI, PI-MgoI, PI-MinI, PI-MkaI, PI-MkaI, PI-MmaI, PI-MshI, PI-MsmI, I-MsoI, PI-MthI, PI-MtuI, PI-MxeI, PI-NpuI, PI-PfuI, PI-RmaI, PI-SpbI, PI-SspI, PI-FacI, PI-MjaI, PI-PhoI, PI-TagI, PI-ThyI, PI-TkoI, PI-TspI, or a variant thereof. The variant is not particularly limited, and may be suitably selected in accordance with the purpose. For example, the variant may have a homology of 80% or more, 90% or more, 95% or more, or 99% or more with the original amino acid sequence.

In a double-stranded circular DNA vector of the present invention, the fusion gene sequence is placed under the control of a promoter which is capable of regulating the expression.

As used herein, a "promoter which is capable of regulating the expression" refers to a promoter which is a regulatory region for gene expression capable of controlling the expression of a gene or the like placed downstream (at the 3'-end side) in a cell having a double-stranded circular DNA vector transduced thereto, and can induce the expression of the subject gene or the like in the cell at any time. A promoter which is capable of regulating the expression can drive induction, for example, in a condition such as in the presence or absence of a specific factor or at a temperature. The kind of a promoter which is capable of regulating the expression is not limited, and may be suitably selected in accordance with the purpose. Examples include thermal induction promoters (lambda PR, lambda PL, and the like), arabinose induction promoters (araBAD promoter and the like), IPTG induction promoters (LAC promoter, LACUV5 promoter, TAC promoter, Trc promoter, LPP promoter, and the like), T7 promoters, low-temperature induction promoters (cspA promoter and the like), Trp promoters, rhamnose induction promoters (rhaT promoter and the like), proU promoter, prpB promoters, phoA promoters, recA promoters, tetA promoters, cadA promoters, and a variant thereof. IPTG induction promoters and arabinose induction promoters are preferable.

As used herein, the phrase "placed under the control of a promoter which is capable of regulating the expression" means that a gene to be expressed or the like is placed in a region downstream of the promoter which is capable of regulating the expression, under the control of the promoter.

The fusion polypeptide may comprise a linker sequence between the protelomerase or an active fragment thereof and the endonuclease or an active fragment thereof. As used herein, a "linker sequence" is a peptide which can be inserted between a protelomerase or an active fragment thereof and an endonuclease or an active fragment thereof in a fusion polypeptide for each fused portion to perform a function of interest. The length of the linker sequence is not limited, and is, for example, 1 amino acid in length or more, 2 amino acids in length or more, 3 amino acids in length or more, 4 amino acids in length or more, 5 amino acids in length or more, 6 amino acids in length or more, 7 amino acids in length or more, 8 amino acids in length or more, 9 amino acids in length or more, 10 amino acids in length or more, 11 amino acids in length or more, 12 amino acids in length or more, 13 amino acids in length or more, 14 amino acids in length or more, 15 amino acids in length or more, 20 amino acids in length or more, 25 amino acids in length or more, 30 amino acids in length or more, 35 amino acids in length or more, 40 amino acids in length or more, 45 amino acids in length or more, 50 amino acids in length or more, 60 amino acids in length or more, 70 amino acids in length or more, 80 amino acids in length or more, 90 amino acids in length or more, 100 amino acids in length or more, 110 amino acids in length or more, 120 amino acids in length or more, 150 amino acids in length or more, 200 amino acids in length or more, 300 amino acids in length or more, 400 amino acids in length or more, or 500 amino acids in length or more, and/or 5000 amino acids in length or less, 4000 amino acids in length or less, 3000 amino acids in length or less, 2000 amino acids in length or less, 1000 amino acids in length or less, 900 amino acids in length or less, 800 amino acids in length or less, 700 amino acids in length or less, or 600 amino acids in length or less, for example, 1 to 500 amino acids in length, 2 to 450 amino acids in length, 3 to 400 amino acids in length, 3 to 350 amino acids in length, 3 to 300 amino acids in length, 3 to 250 amino acids in length, 3 to 200 amino acids in length, 3 to 150 amino acids in length, 4 to 100 amino acids in length, 5 to 50 amino acids in length, 6 to 40 amino acids in length, 7 to 30 amino acids in length, 8 to 20 amino acids in length, 9 to 15 amino acids in length, 10 to 14 amino acids in length, 11 to 13 amino acids in length, or 12 amino acids in length. As the linker sequence, a peptide comprising a lot of amino acids having a relatively small side chain, for example, serine or glycine, is often used.

A pair of protelomerase recognition sequences in a double-stranded circular DNA vector of the present aspect are protelomerase recognition sequences which are recognized by a protelomerase or an active fragment thereof comprised in a fusion polypeptide. Specific examples of the protelomerase recognition sequence include the base sequence (SEQ ID NO: 26) recognized by TelA protelomerase, the base sequence (SEQ ID NO: 27) recognized by *Halomonas virus HAP1-*derived protelomerase, the base sequence (SEQ ID NO: 28) recognized by *Vibrio virus VP882*-derived protelomerase, the base sequence (SEQ ID NO: 29) recognized by TelK protelomerase, the base sequence recognized by *Rhizobium pusense*-derived protelomerase, the base sequence recognized by *Feldmannia species virus-*derived protelomerase, the base sequence recognized by *Vibrio phage vB_VpaM_MAR*-derived protelomerase, the base sequence (SEQ ID NO: 30) recognized by Tel protelomerase, and the base sequence (telRL sequence) (SEQ ID NO: 3) recognized by TelN protelomerase.

An endonuclease recognition sequence in a double-stranded circular DNA vector of the present aspect is an endonuclease recognition sequence which is recognized by an endonuclease or an active fragment thereof comprised in a fusion polypeptide. Examples of the endonuclease recognition sequence include a restriction enzyme recognition sequence, TALEN recognition sequence, ZFN recognition sequence, and CRISPR/Cas9 recognition sequence. In a case where the endonuclease is a homing endonuclease, examples of the homing endonuclease recognition sequence include an I-SceI recognition sequence (SEQ ID NO: 31), I-CeuI recognition sequence (SEQ ID NO: 32), and I-CreI recognition sequence (SEQ ID NO: 33).

The number of endonuclease recognition sequences in a double-stranded circular DNA vector in the present invention may be one or more. For example, the double-stranded circular DNA vector in the present invention may comprise 1, 2, 3, 4, 5, 6, 7, 8, 9, 10, or more endonuclease recognition sequences. However, no endonuclease recognition sequence is comprised in a region comprising a nucleic acid sequence of interest between a pair of protelomerase recognition sequences.

As used herein, the "nucleic acid sequence of interest" is not particularly limited, as long as it is a sequence comprising two or more nucleotides. More specifically, the nucleic acid sequence of interest is not limited to a sequence encoding a protein, a fragment thereof, or an RNA, and may be, for example, a sequence which can function as a nucleic acid drug, or a like sequence, or may be a sequence which can be utilized to transduce a nucleic acid sequence of interest. Specific examples of the nucleic acid sequence include, but are not limited to, gene sequences encoding a protein or a fragment thereof; nucleic acid sequences encoding a noncoding RNA such as siRNA, shRNA, miRNA, lncRNA, or ribozyme; and nucleic acid drugs such as an antisense nucleic acid, aptamer, and bait nucleic acid. In addition, examples of the sequence which may be utilized to transduce a nucleic acid sequence of interest include: a sequence which can be recognized/cleaved by a nuclease such as a restriction enzyme or a genome-editing enzyme; and a multicloning site comprising a sequence which can be recognized/cleaved by two or more nucleases.

As used herein, the "gene sequence of interest" means a gene sequence encoding a protein of interest or a fragment thereof, or an RNA molecule of interest. The protein of interest or a fragment thereof is not particularly limited, and may be suitably selected in accordance with the purpose. Examples include: polypeptides constituting viruses; polypeptides produced by animals, plants, fungi, algae, bacteria, viruses, and the like; and fragments of these polypeptides. These can be used as therapeutic drugs for cells/genes, vaccines, therapeutic drugs for diseases, or the like. The polypeptides produced by animals, plants, fungi, algae, bacteria, viruses, or the like are not particularly limited, and may be suitably selected in accordance with the purpose. Examples include: enzymes such as phytase, amylase, glucosidase, cellulase, lipase, protease, glutaminase, peptidase, oxidase, lactase, xylanase, trypsin, pectinase, and isomerase; antibody-binding proteins such as protein A, protein G, and protein L; antibodies or antibody fragments and complexes (antibody-like molecules) between such a compound other than the antibodies or the antibody fragments as another functional protein and an antibody fragment, examples of which antibodies include a human antibody, humanized antibody, chimeric antibody, llama antibody, alpaca antibody, single-chain antibody, heavy-chain antibody, multivalent antibody, Fab, F(ab'), F(ab')₂, Fc, Fc fuse protein, bispecific antibody, heavy chain (H chain), light chain (L chain), single-chain Fv (scFv), sc(Fv)₂, disulfide-bond Fv (sdFv), diabody, and antibody-like molecule target peptide (microantibody); serum albumins such as human serum albumin; epidermal growth factors such as human epidermal growth factors; insulin; growth hormone; erythropoietin; interferon; blood coagulation factor VIII; granulocyte colony-stimulating factor (G-CSF); granulocyte-macrophage colony-stimulating factor (GM-CSF); thrombopoietin; IL-1; IL-6; tissue plasminogen activator (TPA); urokinase; leptin; stem cell growth factor (SCF); fibroin; fluorescent protein; hepatitis B virus surface antigen; and hirudin.

The nucleic acid sequence of interest such as a gene sequence of interest can comprise a promoter in addition to a nucleic acid sequence of interest (for example, a sequence encoding a protein of interest or a fragment thereof). In addition, the nucleic acid sequence may further comprise a component such as an intron, enhancer, terminator, and/or poly A signal, if desired.

In the double-stranded circular DNA vector of the present aspect, the fusion gene sequence and the endonuclease recognition sequence are placed in the same region between the pair of protelomerase recognition sequences, and the nucleic acid sequence of interest is placed in another region between the pair of protelomerase recognition sequences. The endonuclease recognition sequence may be placed adjacent to one of the protelomerase recognition sequences. A spacer sequence may be placed between the endonuclease recognition sequence and the protelomerase recognition sequence.

In the present aspect, the "spacer sequence" is a sequence which can be inserted between the endonuclease recognition sequence and the protelomerase recognition sequence so that a protelomerase or an active fragment thereof comprised in the fusion polypeptide can recognize the protelomerase recognition sequence, and so that an endonuclease or an active fragment thereof comprised in the fusion polypeptide can recognize the endonuclease recognition sequence. The length of the spacer sequence is not limited, as long as it is one base or more, and is, for example, 1 base or more, 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 14 bases or more, 15 bases or more, 20 bases or more, 25 bases or more, 30 bases or more, 35 bases or more, 40 bases or more, 45 bases or more, 50 bases or more, 55 bases or more, 60 bases or more, 65 bases or more, 70 bases or more, 75 bases or more, 80 bases or more, 85 bases or more, 90 bases or more, 95 bases or more, 100 bases or more, 120 bases or more, 150 bases or more, or 200 bases or more, and/or 1000 bases in length or less, 900 bases in length or less, 800 bases in length or less, 700 bases in length or less, 600 bases in length or less, 500 bases in length or less, 400 bases in length or less, 300 bases in length or less, or 250 bases or less, for example, 1 to 1000 bases in length, 1 to 500 bases in length, 1 to 400 bases in length, 1 to 300 bases in length, 1 to 250 bases in length, 1 to 200 bases in length, 1 to 150 bases in length, 1 to 100 bases in length, 1 to 70 bases in length, 1 to 50 bases in length, 1 to 40 bases in length, 1 to 30 bases in length, 1 to 20 bases in length, 1 to 10 bases in length, 2 to 10 bases in length, 3 to 10 bases in length, 4 to 9 bases in length, 5 to 9 bases in length, or 6 to 8 bases in length.

In one embodiment, the length of the spacer sequence can be determined on the basis of the length of a linker sequence.

A double-stranded circular DNA vector of the present aspect may comprise other sequences in addition to a fusion gene sequence, a pair of protelomerase recognition sequences, at least one endonuclease recognition sequence, and a nucleic acid sequence of interest. The other sequences are not particularly limited, and may be suitably selected in accordance with the purpose. Examples include: an element essential for a common plasmid vector, such as a sequence necessary for intracellular maintenance and replication of a vector, for example, an origin of replication and/or a gene encoding an antibiotic-resistant protein; a sequence encoding an activator or repressor protein, such as a LacI gene or an AraC gene; a cloning site; and an overlapping region for utilizing an In-Fusion cloning system of Clontech Laboratories, Inc., a Gibson Assembly system of New England Biolabs, or the like. In this regard, examples of the gene encoding an antibiotic-resistant protein include; a β-lactamase gene which gives ampicillin resistance (referred to as an "ampR gene" in some cases); an aminoglycoside 3'-phosphotransferase gene which gives kanamycin resistance (referred to as a "kanR gene" in some cases); a tetracycline-efflux transporter gene which gives tetracycline resistance; and a CAT (chloramphenicolacetyltransferase) gene which gives chloramphenicol resistance.

In the present aspect, the other sequences may be placed in any of the following: the same region between a pair of protelomerase recognition sequences and in which a fusion gene sequence and an endonuclease recognition sequence are placed; and the same region between the pair of protelomerase recognition sequences and in which a nucleic acid sequence of interest is placed. The other sequences are more preferably placed between the pair of protelomerase recognition sequences and within the same region as the fusion gene sequence and the endonuclease recognition sequence are placed. For example, all the elements other than the nucleic acid sequence of interest may be placed within the same region as the fusion gene sequence and the endonuclease recognition sequence are placed.

A method of producing a double-stranded circular DNA vector of the present aspect is not particularly limited, and may be suitably selected in accordance with the purpose. Examples include a total synthesis method, a PCR method, and a method to be performed using an In-Fusion cloning system of Clontech Laboratories, Inc., a Gibson Assembly system of New England Biolabs, or the like. The double-stranded circular DNA vector may be produced, for example, on the basis of a commercially available or known vector, or may be produced on the basis of a pUC vector, pET vector, pGEM vector, or the like. In particular, designing based on a plasmid having a pUC-based ori, such as pUC19 vector, is preferable in terms of high efficiency of replication of DNA. A double-stranded circular DNA vector of the present invention may also be produced through adding, to such a commercially available vector, a fusion gene sequence, a pair of protelomerase recognition sequences, at least one endonuclease recognition sequence, and a nucleic acid sequence of interest.

### 1-4. Effects

Inducing the expression of a fusion polypeptide from a double-stranded circular DNA vector of the present aspect allows a protelomerase recognition sequence to be recognized by a protelomerase or an active fragment thereof comprised in the fusion polypeptide, and allows the cleaved ends to be recombined, thereby generating a first linear covalently closed DNA comprising a nucleic acid sequence of interest and a second linear covalently closed DNA comprising a fusion gene sequence and an endonuclease recognition sequence. The second linear covalently closed DNA is cleaved as the endonuclease recognition sequence is recognized by the endonuclease or an active fragment thereof comprised in the fusion polypeptide. This allows the second linear covalently closed DNA to be degraded by an endogenous exonuclease activity, and thus, enables the first linear covalently closed DNA to be produced efficiently.

A double-stranded circular DNA vector of the present invention comprises, in a single DNA vector, all the elements necessary to generate a linear covalently closed DNA in a cell, and thus, makes it possible to easily and efficiently produce a linear covalently closed DNA comprising a nucleic acid sequence of interest.

The present invention also provides a fusion polypeptide comprising a protelomerase or an active fragment thereof at an N-terminal side and an endonuclease or an active fragment thereof at a C-terminal side. Furthermore, the present invention also provides a nucleic acid encoding a fusion polypeptide.

### 2. Transformed cell comprising double-stranded circular DNA vector

### 2-1. Overview

A second aspect of the present invention is a transformed cell. A transformed cell of the present aspect comprises the double-stranded circular DNA vector according to the above first aspect.

### 2-2. Configuration

A transformed cell of the present aspect comprises a double-stranded circular DNA vector as an essential component.

The kind of the transformed cell herein is not limited. The transformed cell may be a cell to which a double-stranded circular DNA vector can be transduced, and in which the double-stranded circular DNA vector can be maintained and/or replicated. Examples include archaea, bacteria, and eukaryotic cells. The bacteria may be *Escherichia coli,* lactic acid bacteria, or *Bacillus subtilis.* Examples of the eukaryotic cells include fungus cells (for example, yeast cells), algal cells, plant cells, protozoan cells, insect cells, nematode cells, fish cells, bird cells (for example, chicken cells), and mammalian cells (for example, mouse cells, chimpanzee cells, and human cells). Among these, cells in which a double-stranded circular DNA vector can be replicated are preferable.

Examples of the *Escherichia coli* include genus *Escherichia.* More specific examples include *Escherichia coli* (for example, an *Escherichia coli* K-12 strain and an *Escherichia coli* B strain).

In addition, a commercially available *Escherichia coli* strain and an *Escherichia coli* strain from a bioresource center may be used. The JM109 strain, DH5 strain, DH5α strain, DH10B strain, NEB10β strain, HST08 strain, HST16CR strain, HB101 strain, W3110 strain, MG1655 strain, BL21 strain, BL21 DE3 strain, and the like may be used. These strains are available from New England Biolabs, Takara Bio Inc., Thermo Fisher Scientific Inc., Toyobo Co., Ltd., ATCC (American Type Culture Collection), NBRP (National Bio Resource Project), and the like.

In addition, strains derived from the above-described *Escherichia coli* strains may be used in the present invention. Examples include a JW3973 strain (available from NBRP) auxotrophic for methionine, JW2806 strain (available from NBRP) auxotrophic for leucine, JW3582 strain (available from NBRP) auxotrophic for cysteine, and ME5305 strain (available from NBRP) auxotrophic for thiamine and histidine.

In one embodiment, a transformed cell of the present aspect expresses an exonuclease. Here, the exonuclease is not particularly limited, as long as it can degrade the DNA cleaved by an endonuclease or an active fragment thereof comprised in a fusion polypeptide from the end of the DNA cleaved. The exonuclease may be, for example, an endogenous exonuclease.

### 2-3. Effects

Inducing the expression of a fusion polypeptide from a double-stranded circular DNA vector in the transformed cell of the present aspect enables a linear covalently closed DNA comprising a nucleic acid sequence of interest to be produced efficiently.

The second linear covalently closed DNA comprising a fusion gene sequence and an endonuclease recognition sequence is cleaved by an endonuclease, and then, can be rapidly degraded by an endogenous exonuclease activity.

### 3. Method of producing linear covalently closed DNA

### 3-1. Overview

A third aspect of the present invention is a method of producing a linear covalently closed DNA. A producing method of the present aspect comprises a culture step, expression-induction step, and DNA extraction step as essential steps, and comprises a separation step and/or a transduction step as an optional step(s). The production method of the present aspect enables a linear covalently closed DNA to be produced easily and efficiently.

### 3-2. Method

Each step in a method of producing a linear covalently closed DNA according to the present aspect will be specifically described below.

### (Transduction step)

The "transduction step" is a step of transducing the double-stranded circular DNA vector of the first aspect to a host cell to producer a transformed cell.

In the present step, a method of transducing a double-stranded circular DNA vector to a host cell is not particularly limited. A transgenic method (transforming method) known in the art, for example, a method described in Green & Sambrook, 2012, Molecular Cloning: A Laboratory Manual Fourth Ed., Cold Spring Harbor Laboratory Press, Cold Spring Harbor, New York may be used. Specific examples include a heat shock method, lipofection method, electroporation method, microinjection method, calcium phosphate method, DEAE dextran method, transduction with a cationic lipid, transduction with a cationic polymer (for example, polyethyleneimine (PEI)), transduction with nanoparticles, transduction with a virus, and particle bombardment.

In the present step, a host cell having a double-stranded circular DNA vector transduced thereto may be suitably selected in a culture medium comprising an antibiotic, on the basis of a gene encoding an antibiotic-resistant protein in the double-stranded circular DNA vector.

### (Culture step)

The "culture step" is a step of culturing a transformed cell. A purpose of the present step is to proliferate transformed cells before the below-described expression-induction step to thereby increase the intracellular copy number of double-stranded circular DNA vectors before proceeding to the below-described expression-induction step.

In the present step, a method of culturing a transformed cell is not particularly limited, and may be suitably selected in accordance with the kind of the transformed cell. A culture method known in the art, for example, a method described in Green & Sambrook, 2012, Molecular Cloning: A Laboratory Manual Fourth Ed., Cold Spring Harbor Laboratory Press, Cold Spring Harbor, New York may be used.

A culture medium for culturing a transformed cell in the present step may be any of a liquid medium and a solid medium. The culture medium may be, for example, a culture medium comprising one or more components selected from: protein enzyme degradation products such as peptone and tryptone; biological extracts such as potato dextrose and yeast extracts; amino acids such as glutamic acid, or salts thereof; saccharides such as glucose, glycerol, and sucrose; and inorganic salts such as sodium chloride, magnesium chloride, and potassium dihydrogenphosphate. Specific examples of the culture medium and the composition include LB culture media (tryptone, yeast extracts, and sodium chloride), YPG culture media (yeast extracts, peptone, and glucose), PD culture media (potato dextrose), and TB culture media (tryptone, yeast extracts, dipotassium hydrogenphosphate, and potassium dihydrogenphosphate).

In a case where a double-stranded circular DNA vector comprises a gene encoding an antibiotic-resistant protein, the present step can be performed in the presence of an antibiotic. Examples of antibiotics include ampicillin, carbenicillin, kanamycin, tetracycline, and chloramphenicol.

Culture conditions in the present step may be suitably selected in accordance with the kind of the transformed cell. A culture in the present step may be performed, for example, at 20 to 42°C, 25 to 40°C, 30 to 38°C, or 35 to 37°C.

Culture time in the present step is not limited, as long as a double-stranded circular DNA vector is produced in a sufficient amount. The culture time may be, for example, 1 hour or more, 2 hours or more, 4 hours or more, 12 hours or more, 24 hours or more, 2 days or more, 3 days or more, or 1 week or more.

In addition, the pH of a culture medium to be used in the present step is not particularly limited, as long as transformed cells can grow. The pH may be, for example, pH 3 or more, pH 4 or more, pH 5 or more, pH 6 or more, pH 7 or more, pH 7.5 or more, pH 8 or more, pH 8.5 or more, pH 9 or more, or pH 9.5 or more, and/or pH 11 or less, pH 10.5 or less, pH 10 or less, pH 9.5 or less, pH 9 or less, pH 8.5 or less, pH 8 or less, or pH 7.5 or less, or may be, for example, pH 3 to 11, pH 4 to 10, pH 5 to 9, or pH 7 to 9, or pH 7.5 to pH 10.5, pH 8 to pH 10, or pH 8.5 to pH 9.5.

### (Expression-induction step)

In the present aspect, the "expression-induction step" is a step of inducing the expression of a fusion polypeptide in the transformed cell after the culture step.

In the present step, a method of inducing the expression of a fusion polypeptide is not particularly limited, and may be suitably selected in accordance with the kind of a promoter which is capable of regulating the expression.

In one embodiment, an agent for inducing the expression is added to a culture medium in the present step after the culture step. For example, in a case where the promoter is an arabinose induction promoter, IPTG induction promoter, or rhamnose induction promoter, it is possible to use, for example, a method in which arabinose, IPTG, or rhamnose as an expression inducer is added to a culture medium comprising a transformed cell, after the culture step.

In the present step, a period of time for culturing a transformed cell in the presence of an agent for inducing the expression is not particularly limited, as long as the expression of a fusion polypeptide is induced sufficiently. The period of time may be, for example, 1 minute or more, 5 minutes or more, 10 minutes or more, 20 minutes or more, 30 minutes or more, 1 hour or more, 2 hours or more, 3 hours or more, 6 hours or more, 12 hours or more, or 24 hours or more.

The pH of a culture medium to be used in the present step may be in a range which enables a fusion polypeptide to function in a cell, that is, a range which enables both a protelomerase and an endonuclease to function, and may be, for example, pH 3 to 11, pH 4 to 10, pH 5 to 9, or pH 7 to 9. For example, a pH range between the optimal pH of a protelomerase and the optimal pH of an endonuclease may be used.

In the present step, a temperature at which a transformed cell is cultured in the presence of an agent for inducing the expression may be, for example, 20°C or more, 25°C or more, 26°C or more, 27°C or more, 28°C or more, 29°C or more, 30°C or more, 31°C or more, 32°C or more, 33°C or more, 34°C or more, 35°C or more, 36°C or more, or 37°C or more, and/or 42°C or less, 40°C or less, 39°C or less, 38°C or less, 37°C or less, 36°C or less, 35°C or less, 34°C or less, 33°C or less, 32°C or less, or 31°C or less, for example,, 30 to 38°C, 32 to 38°C, 33 to 38°C, 34 to 37°C, 35 to 37°C, or 36 to 37°C, or 25 to 35°C, 26 to 34°C, 27 to 33°C, 28 to 32°C, or 29 to 31°C. For example, a temperature range between the optimal temperature of a protelomerase and the optimal temperature of an endonuclease may be used.

In a further embodiment, a transformed cell in the present step may be cultured under a plurality of temperature conditions in the presence of an agent for inducing the expression. Examples of the plurality of temperature conditions include the optimal temperature range for the enzymic activity of each of a protelomerase and an endonuclease. The optimal temperature for the enzymic activity of a protelomerase can be, for example, 26 to 37°C, 27 to 35°C, 28 to 33°C, or 29 to 31°C (for example, 30°C). The optimal temperature for the enzymic activity of a homing endonuclease can be, for example, 30 to 42°C, 32 to 40°C, 34 to 39°C, or 36 to 38°C (for example, 37°C). Culture time under each temperature condition in the present embodiment may be 10 minutes or more, 20 minutes or more, 30 minutes or more, 1 hour or more, 2 hours or more, 3 hours or more, 4 hours or more, 5 hours or more, 6 hours or more, 8 hours or more, 10 hours or more, 12 hours or more, or 24 hours or more. Culture in the present step may be performed in any combination of the above-described temperatures and culture times. For example, it is also possible that culturing at 28 to 32°C for 2 hours or more (for example, 4 hours or more) is followed by culturing at 35°C to 39°C for 2 hours or more (for example, 4 hours or more).

In another embodiment in which a promoter which is capable of regulating the expression is a thermal induction promoter or a low-temperature induction promoter, a heat shock or a low-temperature shock may be applied. The conditions for the heat shock or the low-temperature shock are not particularly limited, as long as the expression of a fusion polypeptide is induced sufficiently by the temperature and time. For example, the heat shock may be performed at 36°C to 45°C, 37°C to 44°C, 38°C to 43°C, 39°C to 42°C, or 40°C to 41°C, for 10 seconds or more, 20 seconds or more, 30 seconds or more, 40 seconds or more, 50 seconds or more, 1 minute or more, 5 minutes or more, 10 minutes or more, 20 minutes or more, 30 minutes or more, or 1 hour or more, and/or 24 hours or less, 12 hours or less, 6 hours or less, 3 hours or less, or 2 hours or less, for example, 10 seconds to 24 hours, 20 seconds to 12 hours, 25 seconds to 6 hours, 30 seconds to 3 hours, 40 seconds to 2 hours, 50 seconds to 1 hour, 1 minute to 30 minutes, or 2 minutes to 5 minutes.

In the present step,a fusion polypeptide is expressed in a transformed cell; a first linear covalently closed DNA comprising a nucleic acid sequence of interest and a second linear covalently closed DNA comprising a fusion gene sequence and an endonuclease recognition sequence are generated; and the second linear covalently closed DNA is cleaved and degraded by an endogenous exonuclease activity.

### (DNA extraction step)

The "DNA extraction step" is a step of extracting DNA from the transformed cell after the expression-induction step.

As a method of extracting DNA in the present step, an ordinary method known in the art may be used. Preparation may be performed, for example, in accordance with a method described in Green & Sambrook, 2012, Molecular Cloning: A Laboratory Manual Fourth Ed., Cold Spring Harbor Laboratory Press, Cold Spring Harbor, New York. In addition, a commercially available DNA extraction kit may be used for the preparation.

The present step allows the first linear covalently closed DNA comprising a nucleic acid sequence of interest to be extracted.

### (Separation step)

The "separation step" is a step of separating the linear covalently closed DNA after the DNA extraction step. A purpose of the present step is to remove a DNA component other than a linear covalently closed DNA comprising a nucleic acid sequence of interest extracted in the DNA extraction step, for example, a genomic DNA fragment, a DNA fragment remaining to be cleaved, and an unnecessary linear covalently closed DNA comprising an endonuclease recognition sequence.

In the present step, a method of separating a linear covalently closed DNA is not particularly limited, and may be suitably selected. Examples include: a method in which purification is performed using a commercially available kit; and a method in which separation is performed using gel extraction, cation chromatography, anion chromatography, size exclusion chromatography, a filter, or an ultrafiltration membrane.

### 3-3. Effects

The method of producing a linear covalently closed DNA according to the present aspect makes it possible to easily and efficiently produce a linear covalently closed DNA. On the basis of the producing method of the present invention, a linear covalently closed DNA can be produced in a large amount with a small number of steps.

### 4. Double-stranded circular DNA vector

### 4-1. Overview

A fourth aspect of the present invention is a double-stranded circular DNA vector. A double-stranded circular DNA vector of the present aspect comprises a protelomerase gene sequence, an endonuclease gene sequence, and an endonuclease recognition sequence in the same region between a pair of protelomerase recognition sequences, and comprises a nucleic acid sequence of interest in another region between the pair of protelomerase recognition sequences.

The double-stranded circular DNA vector of the present aspect makes it possible to easily and efficiently produce a linear covalently closed DNA comprising a nucleic acid sequence of interest.

### 4-2. Configuration

A double-stranded circular DNA vector of the present aspect comprises a protelomerase gene sequence, an endonuclease gene sequence, a pair of protelomerase recognition sequences, at least one endonuclease recognition sequence, and a nucleic acid sequence of interest. In the double-stranded circular DNA vector of the present aspect, the protelomerase gene sequence, the endonuclease gene sequence, and the endonuclease recognition sequence are placed within the same region between the pair of protelomerase recognition sequences, and the nucleic acid sequence of interest is placed within another region between the pair of protelomerase recognition sequences. In addition, the protelomerase gene sequence and the endonuclease gene sequence are placed under the control of a promoter which is capable of regulating the expression.

As used herein, a "protelomerase gene sequence" means a gene sequence encoding a protelomerase or an active fragment thereof. The protelomerase gene sequence may undergo codon optimization in accordance with the codon usage in a cell to which a double-stranded circular DNA vector is transduced.

The protelomerase encoded by a protelomerase gene sequence, or an active fragment of the protelomerase, is not particularly limited to any kind, as long as having a protelomerase activity, and may be those which do not recognize a sequence other than the pair of protelomerase recognition sequences in a double-stranded circular DNA vector, and particularly do not recognize a nucleic acid sequence of interest. In addition, the genomic sequence of a cell to which a double-stranded circular DNA vector is transduced may be those which do not have the protelomerase recognition sequence. For example, the length of the protelomerase recognition sequence which is recognized by the protelomerase or an active fragment thereof may be, but is not limited to, 5 bp or more, 6 bp or more, 7 bp or more, 8 bp or more, 9 bp or more, 10 bp or more, 15 bp or more, 20 bp or more, 25 bp or more, 30 bp or more, 35 bp or more, 40 bp or more, 45 bp or more, 50 bp or more, 55 bp or more, 60 bp or more, 65 bp or more, 70 bp or more, 80 bp or more, or 90 bp or more. Particularly preferable examples of the above-described protelomerases include TelN protelomerase, TelA protelomerase, TelK protelomerase, and a variant thereof. The variant is not particularly limited, and may be suitably selected in accordance with the purpose. For example, the variant may have a homology of 80% or more, 90% or more, 95% or more, or 99% or more with the original amino acid sequence.

As used herein, an "endonuclease gene sequence" means a gene sequence encoding an endonuclease or an active fragment thereof. The endonuclease gene sequence may undergo codon optimization in accordance with the codon usage in a cell to which a double-stranded circular DNA vector is transduced.

The endonuclease or an active fragment thereof encoded by the endonuclease gene sequence is not particularly limited, as long as having an endonuclease activity, and may be those which do not recognize a sequence other than the endonuclease recognition sequence in a double-stranded circular DNA vector, and particularly do not recognize a nucleic acid sequence of interest. In addition, the genomic sequence of a cell to which a double-stranded circular DNA vector is transduced may be those which do not have the endonuclease recognition sequence. The endonuclease or an active fragment thereof may be a restriction enzyme, or may be Nickase. For example, the length of the endonuclease recognition sequence which is recognized by the endonuclease or an active fragment thereof may be, but is not limited to, 4 bp or more, 5 bp or more, 6 bp or more, 7 bp or more, 8 bp or more, 9 bp or more, 10 bp or more, 15 bp or more, 20 bp or more, 25 bp or more, 30 bp or more, 35 bp or more, 40 bp or more, 45 bp or more, 50 bp or more, 60 bp or more, 70 bp or more, 80 bp or more, 90 bp or more, or 100 bp or more.

In a double-stranded circular DNA vector of the present invention, the protelomerase gene sequence and the endonuclease gene sequence are placed under the control of a promoter which is capable of regulating the expression.

In the present aspect, for example, the protelomerase gene sequence and the endonuclease gene sequence may be placed under the control of different promoters which are capable of regulating the expression respectively, or may be placed under the control of the same promoter which is capable of regulating the expression. In a case where the protelomerase gene sequence and the endonuclease gene sequence are placed under the control of the same promoter which is capable of regulating the expression, the protelomerase gene sequence and the endonuclease gene sequence may be placed within the same operon, or a sequence encoding a self-cleaving peptide, and IRES sequence, or the like may be placed between the protelomerase gene sequence and the endonuclease gene sequence.

A pair of protelomerase recognition sequence in a double-stranded circular DNA vector of the present aspect is a protelomerase recognition sequence which is recognized by the protelomerase encoded by a protelomerase gene sequence, or an active fragment of the protelomerase. Specific examples of the protelomerase recognition sequence include the base sequence (SEQ ID NO: 26) recognized by TelA protelomerase, the base sequence (SEQ ID NO: 27) recognized by *Halomonas virus HAP1*-derived protelomerase, the base sequence (SEQ ID NO: 28) recognized by *Vibrio virus* VP882-derived protelomerase, the base sequence (SEQ ID NO: 29) recognized by TelK protelomerase, the base sequence recognized by *Rhizobium pusense-*derived protelomerase, the base sequence recognized by *Feldmannia species virus-*derived protelomerase, the base sequence recognized by *Vibrio phage vB_VpaM_MAR*-derived protelomerase, the base sequence (SEQ ID NO: 30) recognized by Tel protelomerase, and the base sequence (telRL sequence) (SEQ ID NO: 3) recognized by TelN protelomerase.

An endonuclease recognition sequence in a double-stranded circular DNA vector of the present aspect is an endonuclease recognition sequence which is recognized by an endonuclease encoded by the endonuclease gene sequence, or an active fragment of the endonuclease. Examples of the endonuclease recognition sequence include a restriction enzyme recognition sequence, TALEN recognition sequence, ZFN recognition sequence, and CRISPR/Cas9 recognition sequence. In a case where the endonuclease is a homing endonuclease, examples of the homing endonuclease recognition sequence include an I-SceI recognition sequence (SEQ ID NO: 31), I-CeuI recognition sequence (SEQ ID NO: 32), and I-CreI recognition sequence (SEQ ID NO: 33).

In the double-stranded circular DNA vector of the present aspect, the protelomerase gene sequence, the endonuclease gene sequence, and the endonuclease recognition sequence are placed within the same region between the pair of protelomerase recognition sequences, and the nucleic acid sequence of interest is placed within another region between the pair of protelomerase recognition sequences. The endonuclease recognition sequence may be placed adjacent to one of the protelomerase recognition sequences. A spacer sequence may be placed between the endonuclease recognition sequence and the protelomerase recognition sequence.

In the present aspect, the "spacer sequence" is a sequence which can be inserted between the endonuclease recognition sequence and the protelomerase recognition sequence so that a protelomerase or an active fragment thereof can recognize the protelomerase recognition sequence, and so that an endonuclease or an active fragment thereof can recognize the endonuclease recognition sequence. The length of the spacer sequence is not limited, as long as it is one base or more, and is, for example, 1 base or more, 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 14 bases or more, 15 bases or more, 20 bases or more, 25 bases or more, 30 bases or more, 35 bases or more, 40 bases or more, 45 bases or more, 50 bases or more, 55 bases or more, 60 bases or more, 65 bases or more, 70 bases or more, 75 bases or more, 80 bases or more, 85 bases or more, 90 bases or more, 95 bases or more, 100 bases or more, 120 bases or more, 150 bases or more, or 200 bases or more, and/or 1000 bases in length or less, 900 bases in length or less, 800 bases in length or less, 700 bases in length or less, 600 bases in length or less, 500 bases in length or less, 400 bases in length or less, 300 bases in length or less, or 250 bases or less, for example, 1 to 1000 bases in length, 1 to 500 bases in length, 1 to 400 bases in length, 1 to 300 bases in length, 1 to 250 bases in length, 1 to 200 bases in length, 1 to 150 bases in length, 1 to 100 bases in length, 1 to 70 bases in length, 1 to 50 bases in length, 1 to 40 bases in length, 1 to 30 bases in length, 1 to 20 bases in length, 1 to 10 bases in length, 2 to 10 bases in length, 3 to 10 bases in length, 4 to 9 bases in length, 5 to 9 bases in length, or 6 to 8 bases in length.

A double-stranded circular DNA vector of the present aspect may comprise other sequences in addition to a protelomerase gene sequence, an endonuclease gene sequence, a pair of protelomerase recognition sequences, at least one endonuclease recognition sequence, and a nucleic acid sequence of interest. The other sequences are not particularly limited, and may be suitably selected in accordance with the purpose. Examples include: an element essential for a common plasmid vector, such as a sequence necessary for intracellular maintenance and replication of a vector, for example, an origin of replication and/or a gene encoding an antibiotic-resistant protein; a sequence encoding an activator or repressor protein, such as a LacI gene or an AraC gene; a cloning site; and an overlapping region for utilizing an In-Fusion cloning system of Clontech Laboratories, Inc., a Gibson Assembly system of New England Biolabs, or the like. In this regard, examples of the gene encoding an antibiotic-resistant protein include; a β-lactamase gene which gives ampicillin resistance (ampR gene); an aminoglycoside 3'-phosphotransferase gene which gives kanamycin resistance (kanR gene); a tetracycline-efflux transporter gene which gives tetracycline resistance; and a CAT (chloramphenicolacetyltransferase) gene which gives chloramphenicol resistance.

In the present aspect, the other sequences may be placed between a pair of protelomerase recognition sequences in any of the following: the same region as a protelomerase gene sequence, an endonuclease gene sequence, and an endonuclease recognition sequence; and the same region as a nucleic acid sequence of interest is placed between the pair of protelomerase recognition sequences. The other sequence is more preferably placed between the pair of protelomerase recognition sequences within the same region as the protelomerase gene sequence, the endonuclease gene sequence, and the endonuclease recognition sequence. For example, all the elements other than the nucleic acid sequence of interest may be placed within the same region as the protelomerase gene sequence, the endonuclease gene sequence, and the endonuclease recognition sequence are placed.

A method of producing a double-stranded circular DNA vector of the present aspect is not particularly limited, and may be suitably selected in accordance with the purpose. Examples include a total synthesis method, a PCR method, and a method to be performed using an In-Fusion cloning system of Clontech Laboratories, Inc., a Gibson Assembly system of New England Biolabs, or the like. The double-stranded circular DNA vector may be produced, for example, on the basis of a commercially available or known vector, or may be produced on the basis of a pUC vector, pET vector, pGEM vector, or the like. In particular, designing based on a plasmid having a pUC-based ori, such as pUC19 vector, is preferable in terms of high efficiency of replication of DNA. A double-stranded circular DNA vector of the present invention may be produced through adding, to such a commercially available vector, a protelomerase gene sequence, an endonuclease gene sequence, a pair of protelomerase recognition sequences, at least one endonuclease recognition sequence, and a nucleic acid sequence of interest.

In an embodiment of the present aspect, the protelomerase gene sequence and the endonuclease gene sequence encode a fusion polypeptide containing the protelomerase or an active fragment thereof and the endonuclease or an active fragment thereof. The present embodiment corresponds to the above-described first aspect, and the gene sequence encoding the fusion polypeptide in the present embodiment corresponds to the fusion gene sequence in the first aspect.

### 4-3. Effects

Inducing the expression of an endonuclease or an active fragment thereof and a protelomerase or an active fragment thereof from a double-stranded circular DNA vector of the present aspect allows a protelomerase recognition sequence to be recognized by a protelomerase or an active fragment thereof, and allows the cleaved ends to be recombined, thereby generating a first linear covalently closed DNA comprising a nucleic acid sequence of interest and a second linear covalently closed DNA comprising a protelomerase gene sequence, an endonuclease gene sequence, and an endonuclease recognition sequence. The second linear covalently closed DNA is cleaved as the endonuclease recognition sequence is recognized by the endonuclease or an active fragment thereof. This allows the second linear covalently closed DNA to be degraded by an endogenous exonuclease activity, and thus, enables the first linear covalently closed DNA to be produced efficiently.

### 5. Transformed cell comprising double-stranded circular DNA vector

### 5-1. Overview

A fifth aspect of the present invention is a transformed cell. A transformed cell of the present aspect comprises the double-stranded circular DNA vector described in the above fourth aspect.

### 5-2. Configuration

A transformed cell of the present aspect comprises a double-stranded circular DNA vector as an essential component.

In one embodiment, a transformed cell of the present aspect expresses an exonuclease. Here, the exonuclease is not particularly limited, as long as it can degrade the DNA cleaved by an endonuclease encoded by an endonuclease gene sequence, or an active fragment of the endonuclease from the ends. The exonuclease may be, for example, an endogenous exonuclease.

### 5-3. Effects

Inducing the expression of an endonuclease or an active fragment thereof and a protelomerase or an active fragment thereof from a double-stranded circular DNA vector in the transformed cell of the present aspect enables a linear covalently closed DNA comprising a nucleic acid sequence of interest to be produced efficiently.

The second linear covalently closed DNA comprising a protelomerase gene sequence, an endonuclease gene sequence, and an endonuclease recognition sequence is cleaved by an endonuclease, and then, can be rapidly degraded by an endogenous exonuclease activity.

### 6. Method of producing linear covalently closed DNA

### 6-1. Overview

A sixth aspect of the present invention is a method of producing a linear covalently closed DNA. A producing method of the present aspect comprises a culture step, expression-induction step, and DNA extraction step as essential steps, and comprises a separation step and/or a transduction step as an optional step(s). The producing step of the present aspect enables a linear covalently closed DNA to be produced easily and efficiently.

### 6-2. Method

Each step in a method of producing a linear covalently closed DNA according to the present aspect will be specifically described below.

### (Transduction step)

The "transduction step" is a step of transducing the double-stranded circular DNA vector of the first aspect to a host cell to produce a transformed cell. The constitution of the present step is in accordance with the description of the third aspect, and thus, is omitted here.

### (Culture step)

The "culture step" is a step of culturing a transformed cell. A purpose of the present step is to proliferate transformed cells before the below-described expression-induction step to thereby increase the intracellular copy number of double-stranded circular DNA vectors before proceeding to the below-described expression-induction step. The constitution of the present step is in accordance with the description of the third aspect, and thus, is omitted here.

### (Expression-induction step)

In the present aspect, the "expression-induction step" is a step of inducing the expression of an endonuclease or an active fragment thereof and a protelomerase or an active fragment thereof in the transformed cell after the culture step.

In the present step, a method of inducing the expression of an endonuclease or an active fragment thereof and a protelomerase or an active fragment thereof is not particularly limited, and may be suitably selected in accordance with the kind of a promoter which is capable of regulating the expression.

In one embodiment, an agent for inducing the expression is added to a culture medium in the present step after the culture step. In a case where the promoter is an arabinose induction promoter, IPTG induction promoter, or rhamnose induction promoter, it is possible to use, for example, a method in which arabinose, IPTG, or rhamnose as an agent for inducing the expression is added to a culture medium comprising a transformed cell after the culture step.

In the present step, a period of time for culturing a transformed cell in the presence of an agent for inducing the expression is not particularly limited, as long as the expression of an endonuclease or an active fragment thereof and a protelomerase or an active fragment thereof is induced sufficiently. The period of time may be, for example, 1 minute or more, 5 minutes or more, 10 minutes or more, 20 minutes or more, 30 minutes or more, 1 hour or more, 2 hours or more, 3 hours or more, 6 hours or more, 12 hours or more, or 24 hours or more.

The pH of a culture medium to be used in the present step may be in a range which enables an endonuclease or an active fragment thereof and/or a protelomerase or an active fragment thereof to function in a cell, and may be, for example, pH 3 to 11, pH 4 to 10, pH 5 to 9, or pH 7 to 9. For example, a pH range between the optimal pH of a protelomerase and the optimal pH of an endonuclease can be used.

In the present step, a temperature at which a transformed cell is cultured in the presence of an agent for inducing the expression may be, for example, 20°C or more, 25°C or more, 28°C or more, 30°C or more, or 31°C or more, and/or 42°C or less, 40°C or less, 39°C or less, 38°C or less, 37°C or less, 36°C or less, 35°C or less, 34°C or less, 33°C or less, 32°C or less, or 31°C or less, for example, 26 to 38°C, 27 to 37°C, 28 to 36°C, 29 to 35°C, 30 to 34°C, 30 to 33°C, 30 to 32°C, or 30 to 31°C. For example, a temperature range between the optimal temperature of a protelomerase and the optimal temperature of an endonuclease may be used.

In a further embodiment, a transformed cell in the present step may be cultured under a plurality of temperature conditions in the presence of an agent for inducing the expression. The plurality of temperature conditions may be selected from the optimal temperature range for the enzymic activity of each of a protelomerase and an endonuclease. The optimal temperature for the enzymic activity of a protelomerase can be, for example, 26 to 37°C, 27 to 35°C, 28 to 33°C, or 29 to 31°C (for example, 30°C). The optimal temperature for the enzymic activity of a homing endonuclease can be, for example, 30 to 42°C, 32 to 40°C, 34 to 39°C, or 36 to 38°C (for example, 37°C). A culture time under each temperature condition in the present embodiment may be 10 minutes or more, 20 minutes or more, 30 minutes or more, 1 hour or more, 2 hours or more, 3 hours or more, 6 hours or more, 12 hours or more, or 24 hours or more.

In another embodiment in which a promoter which is capable of regulating the expression is a thermal induction promoter or a low-temperature induction promoter, a heat shock or a low-temperature shock may be applied. The conditions for the heat shock or the low-temperature shock are not particularly limited, as long as the expression of an endonuclease or an active fragment thereof and a protelomerase or an active fragment thereof is induced sufficiently by the temperature and time. For example, the heat shock may be performed at 36°C to 45°C, 37°C to 44°C, 38°C to 43°C, 39°C to 42°C, or 40°C to 41°C, for 10 seconds or more, 20 seconds or more, 30 seconds or more, 40 seconds or more, 50 seconds or more, 1 minute or more, 5 minutes or more, 10 minutes or more, 20 minutes or more, 30 minutes or more, or 1 hour or more, and/or 24 hours or less, 12 hours or less, 6 hours or less, 3 hours or less, or 2 hours or less, for example, 10 seconds to 24 hours, 20 seconds to 12 hours, 25 seconds to 6 hours, 30 seconds to 3 hours, 40 seconds to 2 hours, 50 seconds to 1 hour, 1 minute to 30 minutes, or 2 minutes to 5 minutes.

In the present step, an endonuclease or an active fragment thereof and a protelomerase or an active fragment thereof are expressed in a transformed cell; a first linear covalently closed DNA comprising a nucleic acid sequence of interest and a second linear covalently closed DNA comprising a protelomerase gene sequence, an endonuclease gene sequence, and an endonuclease recognition sequence are generated; and the second linear covalently closed DNA is cleaved and degraded by an endogenous exonuclease activity.

### (DNA extraction step)

The "DNA extraction step" is a step of extracting DNA from the transformed cell after the expression-induction step. The constitution of the present step is in accordance with the description of the third aspect, and thus, is omitted here.

### (Separation step)

The "separation step" is a step of separating the linear covalently closed DNA after the DNA extraction step. A purpose of the present step is to remove a DNA component other than a linear covalently closed DNA extracted in the DNA extraction step, and comprising a nucleic acid sequence of interest, examples of which DNA component include a genomic DNA fragment, a DNA fragment remaining to be cleaved, and an unnecessary linear covalently closed DNA comprising an endonuclease recognition sequence. The constitution of the present step is in accordance with the description of the third aspect, and thus, is omitted here.

### 6-3. Effects

The method of producing a linear covalently closed DNA according to the present aspect makes it possible to easily and efficiently produce a linear covalently closed DNA. On the basis of the producing method of the present aspect, a linear covalently closed DNA can be produced in a large scale with a small number of steps.

### Examples

The present invention will be specifically described below with reference to Examples. In this regard, these Example are intended to explain the present invention, and do not limit the scope of the present invention.

### <Example 1: Construction of vectors for producing linear covalently closed DNA>

### (Purpose)

The purpose is to produce a vector for producing a linear covalently closed DNA.

### (Method and results)

In the below-described Examples, a nucleic acid was manipulated on the basis of a recombinant DNA technology with reference to the following two documents: Molecular Cloning 2nd Edition (Cold Spring Harbor Laboratory Press, 1989); and Current Protocols in Molecular Biology (Greene Publishing Associates and Wiley-Interscience).

In addition, Prime STAR MAX DNA Polymerase (manufactured by Takara Bio Inc.) and the like were used for a PCR in the below-described Examples. The reaction conditions were in accordance with a method described in the attached manual.

### (1) Production of nucleic acid fragment

A nucleic acid fragment to be used to construct a vector for producing a linear covalently closed DNA was produced as follows.

### (a) Nucleic acid encoding fusion polypeptide (SEQ ID NO: 1)

A nucleic acid encoding a fusion polypeptide (SEQ ID NO: 1) was produced through a PCR using a primer 1 (forward, SEQ ID NO: 14) and a primer 4 (reverse, SEQ ID NO: 17), in which fusion polypeptide, TelN protelomerase (a protelomerase derived from *Escherichia virus N15*)*,* a linker sequence (an amino acid sequence encoded by the base sequence of SEQ ID NO: 34), and I-SceI homing endonuclease (a homing endonuclease derived from *Saccharomyces cerevisiae*) were linked in this order from the N-terminal side.

### (b) Nucleic acid (SEQ ID NO: 2) encoding TelN protelomerase

A nucleic acid encoding TelN protelomerase (SEQ ID NO: 2) was produced through a PCR using the primer 1 (forward, SEQ ID NO: 14) and a primer 2 (reverse, SEQ ID NO: 15), and using, as a template, the above-described nucleic acid (SEQ ID NO: 1) encoding a fusion polypeptide.

### (c) Pair of protelomerase recognition sequences (telRL sequence, SEQ ID NO: 3)

A nucleic acid having a pair of protelomerase recognition sequences (the telRL sequence, SEQ ID NO: 3) was produced through total synthesis.

### (d) Nucleic acid (SEQ ID NO: 4) encoding I-SceI homing endonuclease

A nucleic acid encoding I-SceI homing endonuclease (SEQ ID NO: 4) derived from *Saccharomyces cerevisiae* was produced through a PCR using a primer 3 (forward, SEQ ID NO: 16) and the primer 4 (reverse, SEQ ID NO: 17), and using, as a template, the above-described nucleic acid (SEQ ID NO: 1) encoding a fusion polypeptide.

### (e) Sequences comprising telRL sequence and I-SceI recognition sequence (SEQ ID NOs: 5 to 9)

The sequences comprising a telRL sequence and an I-SceI recognition sequence (SEQ ID NOs: 5 to 9) were produced through total synthesis. SEQ ID NO: 5 is a sequence having a telRL sequence and an I-SceI recognition sequence which are adjacent to each other without a spacer sequence therebetween. SEQ ID NOs: 6, 7, 8, and 9 comprise T2, T4, T6, and T8 respectively which are each a spacer sequence between the telRL sequence and the I-SceI recognition sequence, with T0 representing "no spacer".

### (f) AraC gene sequence under the control of promoter (SEQ ID NO: 11)

An AraC gene sequence under the control of a promoter (SEQ ID NO: 11) was produced through a PCR using a primer 5 (forward, SEQ ID NO: 18) and a primer 6 (reverse, SEQ ID NO: 19).

### (g) Arabinose induction promoter sequence (SEQ ID NO: 12)

An Arabinose induction promoter sequence (SEQ ID NO: 12) was produced through total synthesis.

### (h) Arabinose induction promoter sequence comprising regulatory sequence (SEQ ID NO: 13)

An Arabinose induction promoter sequence comprising a regulatory sequence (SEQ ID NO: 13) was produced through a PCR using a primer 7 (forward, SEQ ID NO: 20) and a primer 8 (reverse, SEQ ID NO: 21).

### (2) Production of control vector

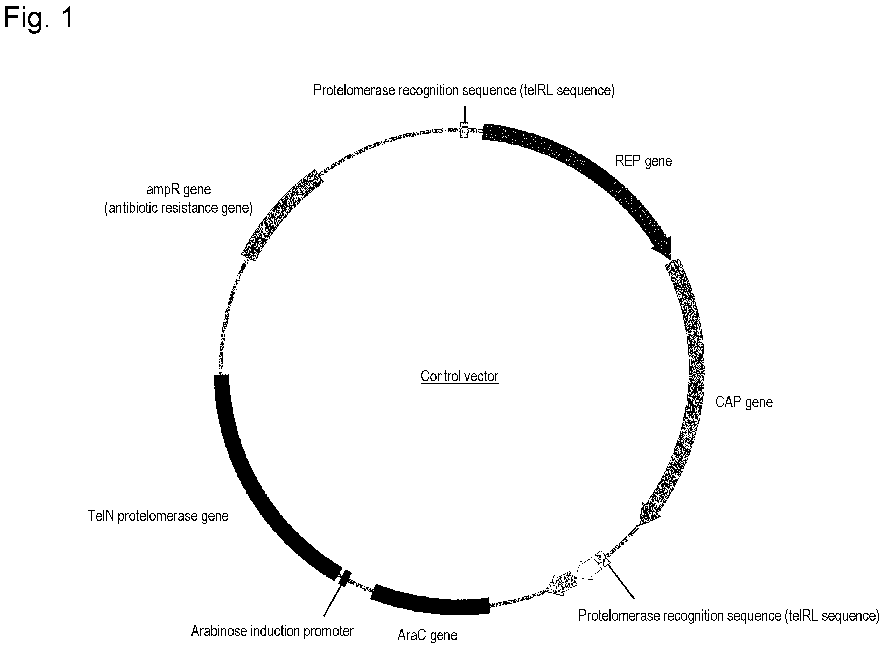

A vector for expresssing TelN protelomerase under the control of an arabinose induction promoter (the vector is hereinafter referred to as a "control vector") was produced as a vector for producing a linear covalently closed DNA (Figure 1).

A method of producing the control vector was as follows. First, the telRL sequence in (c) above was inserted into the EcoRV site and the SnaBI site of a pRC2-mi342 vector (manufactured by Takara Bio Inc.). Then, (b), (f), and (g) above were inserted into the SmaI site of this vector.

In the control vector, the Rep gene (SEQ ID NO: 22) and the Cap gene (SEQ ID NO: 23) were placed between a pair of protelomerase recognition sequences (telRL sequences) (Figure 1). In this regard, the Rep gene and the Cap gene each encode a packaging protein of an adeno-associated virus.

### (3) Production of separate expression vector

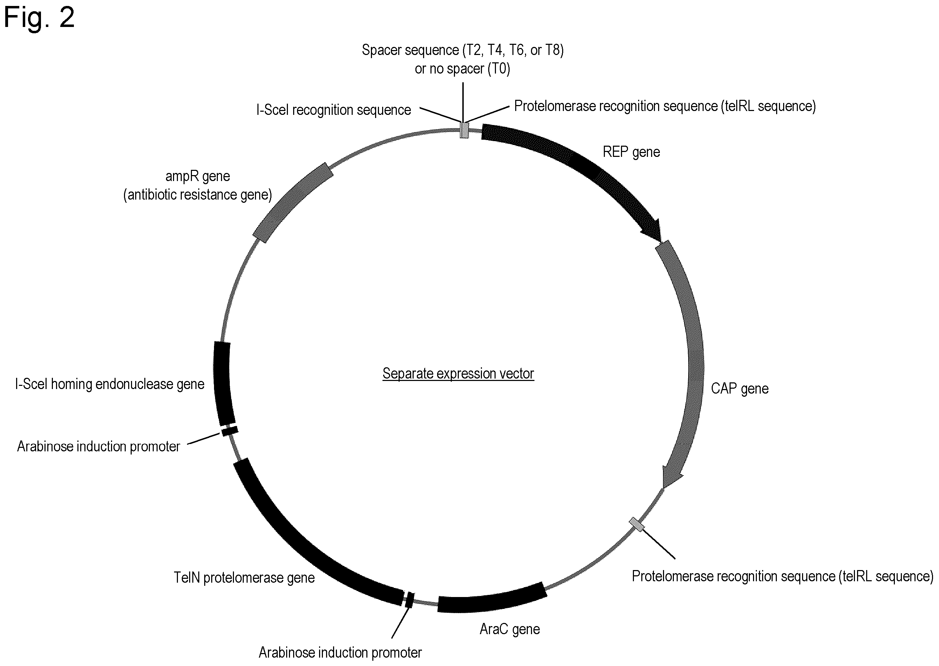

A vector comprising TelN protelomerase gene and I-SceI homing endonuclease gene (the vector is hereinafter referred to as a "separate expression vector") was produced as a vector for producing a linear covalently closed DNA (Figure 2).

A method of producing the separate expression vector was as follows. First, the telRL sequence in (c) above was inserted into the SnaBI site of a pRC2-mi342 vector (manufactured by Takara Bio Inc.). Then, (b), (d), (f), (g), and (h) above were inserted into the SmaI site of this vector. In the EcoRV site of the resulting vector, the five sequences (SEQ ID NOs: 5 to 9) comprising the telRL sequence and the I-SceI recognition sequence in (e) above were inserted to produce a separate expression vector (T0), separate expression vector (T2), separate expression vector (T4), separate expression vector (T6), and separate expression vector (T8) (Figure 2).

The separate expression vectors were designed so that the I-SceI homing endonuclease and the TelN protelomerase could each be separately expressed under the control of an arabinose induction promoter. In addition, the Rep gene and the Cap gene were placed between a pair of protelomerase recognition sequences (telRL sequences) (Figure 2).

### <Example 2: Construction of fusion expression vector>

### (Purpose)

The purpose is to produce, as a vector for producing a linear covalently closed DNA, a vector comprising a gene encoding a fusion polypeptide of TelN protelomerase and I-SceI homing endonuclease (the vector is hereinafter referred to as a "fusion expression vector").

### (Method and results)

### (1) Structure prediction

The conformation of TelN protelomerase was predicted using the following method.

The crystal structure of each of TelK protelomerase (having a sequence homology of approximately 83%) and TelA protelomerase (having a sequence homology of approximately 8%) as enzymes which catalyze the DNA cleaving/recombining reaction in the same manner as TelN protelomerase was partly determined (PDBID: 2V6E, 4DWP, 4E0G, and the like), but for neither protelomerase, the full-length conformation comprising the C-terminal side was determined. In order that the DNA cleaving/recombining reaction and the degradation of an unnecessary linear covalently closed DNA can be simultaneously performed efficiently using a fusion polypeptide of TelN protelomerase and I-SceI homing endonuclease, the placement of the I-SceI homing endonuclease and the TelN protelomerase in the fusion polypeptide (the fusion at the N-terminal side or the C-terminal side) and the respective relative positions with respect to the I-SceI homing endonuclease recognition sequence and the TelN protelomerase recognition sequence (telRL sequence) are important. Accordingly, the full-length conformation of TelN protelomerase was predicted using high-accuracy protein conformation prediction software ColabFold (Mirdita M, et al., Nature Methods (2022) doi: 10.1038/s41592-022-01488-1).

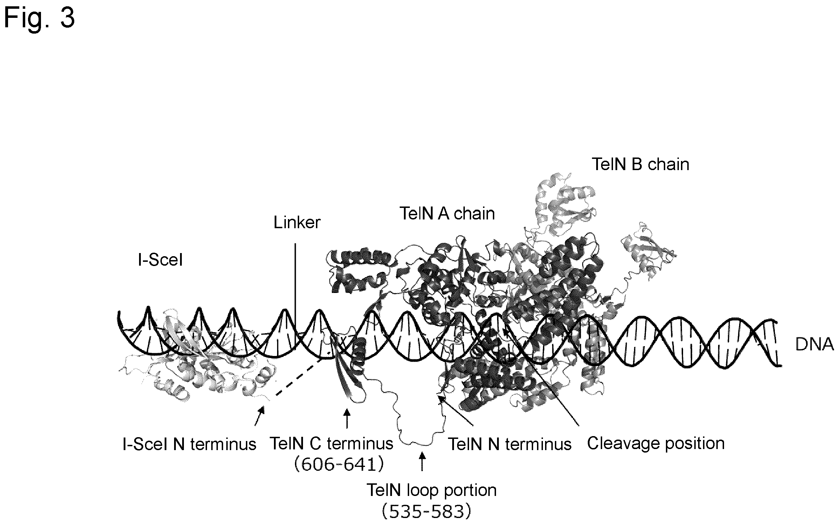

The predicted structure of TelN protelomerase has revealed that the N-terminal side of TelN protelomerase was located near the center of the DNA recognition sequence (telRL sequence), but on the other hand, the C-terminal side (positions 606 to 641) was folded and located far from the DNA recognition sequence, and was able to take various relative configurations with respect to the DNA. This is because a loop region (positions 535 to 583) predicted between the N-terminal side and the C-terminal side was flexible, thus enabling the C-terminal side bound via the loop region to take various configurations even in a case where the N-terminal side was bound to the DNA (Figure 3). Accordingly, when the fusion polypeptide (PH type fusion polypeptide in Example 5) in which the N-terminal side of the I-SceI endonuclease was fused to the C-terminal side of the TelN protelomerase was compared with the fusion polypeptide placed oppositely (HP type fusion polypeptide in Example 5), it was predicted that the activities of both polypeptides could function more efficiently. The placement of the PH type fusion polypeptide and the DNA recognition sequence is illustrated in Figure 3.

### (2) Production of fusion expression vector

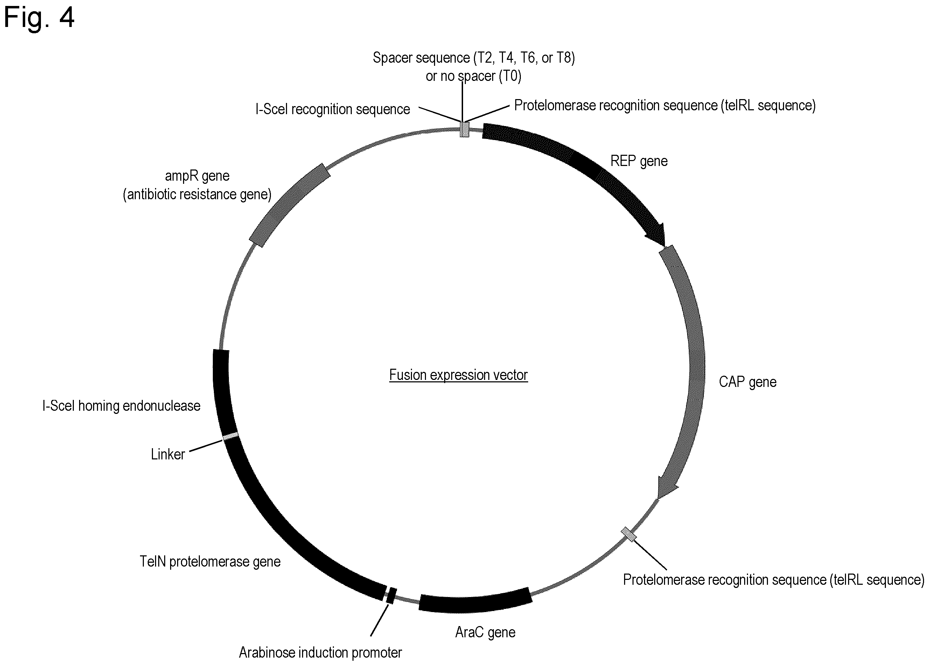

The method of producing a fusion expression vector was as follows. First, the telRL sequence in (c) in Example 1 was inserted into the SnaBI site of a pRC2-mi342 vector (manufactured by Takara Bio Inc.). Then, (a), (f), and (g) in Example 1 were inserted into the SmaI site of this vector. In the EcoRV site of the resulting vector, the five sequences (SEQ ID NOs: 5 to 9) comprising the telRL sequence and the I-SceI recognition sequence in (e) in Example 1 were inserted to produce a fusion expression vector (T0), fusion expression vector (T2), fusion expression vector (T4), fusion expression vector (T6), and fusion expression vector (T8) (Figure 4).

The fusion expression vector was designed so that a fusion polypeptide of the I-SceI homing endonuclease and the TelN protelomerase could be expressed under the control of an arabinose induction promoter. In addition, the Rep gene and the Cap gene were placed between a pair of protelomerase recognition sequences (telRL sequences) (Figure 4).

### <Example 3: Production of linear covalently closed DNA>

### (Purpose)

The purpose is to transform *Escherichia coli* with a vector for producing a linear covalently closed DNA produced in Example 1, and thereby produce a linear covalently closed DNA. The purpose is also to evaluate the amount of production of a linear covalently closed DNA.

### (Method and results)

### (1) Transformation

*Escherichia coli* was transformed with each of the vectors produced in Example 1: a control vector, separate expression vector (T0), separate expression vector (T2), separate expression vector (T4), separate expression vector (T6), separate expression vector (T8), fusion expression vector (T0), fusion expression vector (T2), fusion expression vector (T4), fusion expression vector (T6), and fusion expression vector (T8).

A competent cell solution of an *Escherichia coli DH10B* strain, in an amount of 25 µL, and a solution comprising 100 pg of each vector were mixed and left to stand on ice for 30 minutes. After standing for 30 minutes, the resulting solution was heat-treated at 42°C for 45 seconds, and left to stand on ice for 2 minutes. Then, 225 µL of an SOC culture medium was added. The *Escherichia coli* was applied to an LB agar culture medium (1% tryptone, 0.5% dry yeast extract, 1% sodium chloride, and 0.005% carbenicillin disodium (manufactured by Nacalai Tesque, Inc.)). A strain which grew in a stationary culture performed at 37°C for 1 day was selected to obtain *Escherichia coli* having a vector transduced thereto.

### (2) Culture

The resulting *Escherichia coli* was inoculated into 2 mL of a Plusgrow II medium (4% Plusgrow II (manufactured by Nacalai Tesque, Inc.) and 0.005% carbenicillin disodium). The resulting mixture was cultured with shaking at 37°C for 6 hours to obtain a preculture liquid. Into 100 mL of a Plusgrow II medium (4%Plusgrow II and 0.005% carbenicillin disodium) in a flask, 200 µL of the preculture liquid was inoculated. The resulting mixture was cultured with shaking at 37°C for 16 hours.

### (3) Expression induction

To the resulting culture liquid, 100 mL of a Plusgrow II medium (4% Plusgrow II and 0.005% carbenicillin disodium) having pH 7.0 and 2 mL of an arabinose solution (10% arabinose) were added. The resulting mixture was cultured with shaking at 30°C or 37°C for 6 hours. Then, bacterial bodies were collected through centrifugation.

### (4) DNA extraction

From 1 mL of the culture liquid collected, DNA was extracted using a QIA prep Spin Miniprep Kit (manufactured by QIAGEN N.V.).

### (5) Evaluation of total DNA yield

Table 1 below shows the results obtained by calculating the total DNA yield obtained from 200 mL of the culture liquid based on a measurement made using Nanodrop (manufactured by Thermo Fisher Scientific Inc.).

**[Table 1]**

| Table 1: Measurement results of total DNA yield | | | |
|---|---|---|---|
| Vector for producing linear covalently closed DNA | | Total DNA yield (mg) from 200 mL of culture liquid | |
| | | Expression-induction culture temperature: 30°C | Expression-induction culture temperature: 37°C |
| Separate expression vector | T0 | 2.54 | 2.87 |
| | T2 | 4.78 | 3.96 |
| | T4 | 4.15 | 3.44 |
| | T6 | 3.30 | 3.91 |
| | T8 | 4.81 | 2.39 |
| Fusion expression vector | T0 | 3.62 | 2.54 |
| | T2 | 4.54 | 1.55 |
| | T4 | 4.01 | 3.45 |
| | T6 | 4.05 | 2.72 |
| | T8 | 2.64 | 1.97 |
| Control vector | | 6.26 | 3.23 |

### (6) Evaluation of linear covalently closed DNA

A linear covalently closed DNA was evaluated using electrophoresis. Specifically, the DNA solution obtained in (4) above was adjusted to 2 to 10 ng/µL, and analyzed using a microchip electrophoresis system MultiNA for analyzing DNA/RNA (manufactured by Shimadzu Corporation).

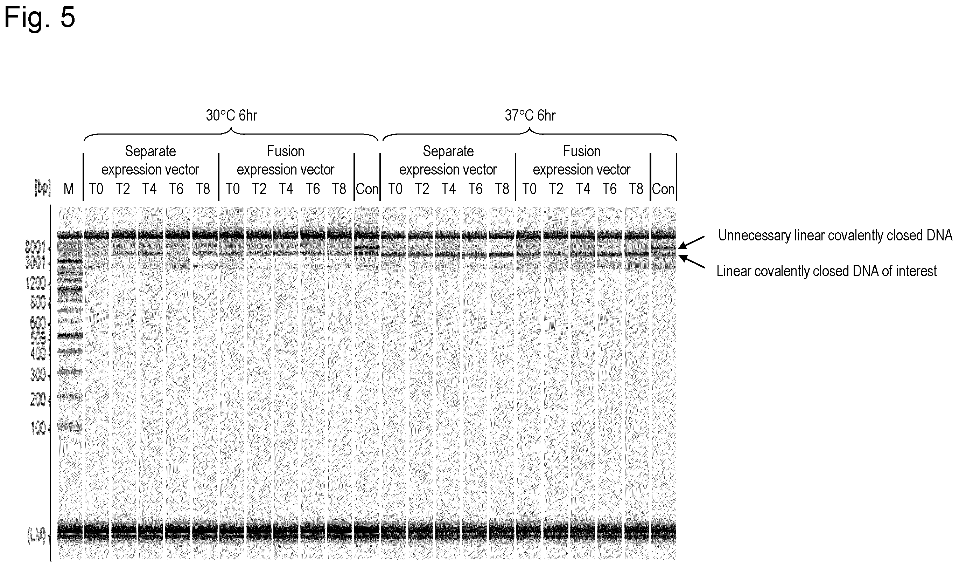

Figure 5 illustrates the electrophoresis results obtained using MultiNA. In Figure 5, a linear covalently closed DNA comprising a nucleic acid encoding a packaging protein is illustrated as a "linear covalently closed DNA of interest", and a linear covalently closed DNA derived from a vector sequence excluding the nucleic acid encoding a packaging protein is illustrated as an "unnecessary linear covalently closed DNA". In the diagram, the left-side image shows the shaking culture performed at an expression-induction culture temperature of 30°C for 6 hours, and the right-side image shows a sample cultured with shaking at an expression-induction culture temperature of 37°C for 6 hours.

Furthermore, the residual ratio of the unnecessary linear covalently closed DNA was calculated from the molar concentrations of the linear covalently closed DNA of interest and the unnecessary linear covalently closed DNA. The formula in accordance with which the residual ratio was calculated is shown below. Residual ratio (%) = (number of moles of unnecessary linear covalently closed DNA)/(number of moles of linear covalently closed DNA of interest) × 100

Table 2 below shows the results obtained by calculating the residual ratio under each condition.

**[Table 2]**

| Table 2: Residual ratio of unnecessary linear covalently closed DNA | | | |
|---|---|---|---|
| Vector for producing linear covalently closed DNA | | Residual ratio (%) of unnecessary linear covalently closed DNA | |
| | | Expression-induction culture temperature: 30°C | Expression-induction culture temperature: 37°C |
| Separate expression vector | T0 | 49.2 | 12.6 |
| | T2 | 65.2 | 9.1 |
| | T4 | 49.1 | 11.5 |
| | T6 | 76.9 | 4.9 |
| | T8 | 66.5 | 1.9 |
| Fusion expression vector | T0 | 19.0 | 25.1 |
| | T2 | 50.8 | 22.5 |
| | T4 | 44.7 | 25.4 |
| | T6 | 48.5 | 9.7 |
| | T8 | 56.6 | 13.4 |
| Control vector | | 115.8 | 90.7 |

According to the results shown in Figure 5 and Table 2, the residual ratio of an unnecessary linear covalently closed DNA from the control vector was 115.8% (30°C) and 90.7% (37°C). On the other hand, the residual ratio of an unnecessary linear covalently closed DNA from the separate expression vector and the fusion expression vector decreased significantly. These results suggest that, in both the separate expression vector and the fusion expression vector, the homing endonuclease recognition sequence was cleaved by the I-SceI homing nuclease, and degradation by the endogenous exonuclease in the *Escherichia coli* body was caused from the cleaved end.

### <Example 4: Study on expression-induction condition>

### (Purpose)

The purpose is to study a culture condition for producing a linear covalently closed DNA. Specifically, the purpose is to study a pH and a temperature condition for culturing a transformed *Escherichia coli.*

### (Method and results)

### (1) Production of linear covalently closed DNA

*Escherichia coli* was transformed using the same method as in "(1) Transformation" in Example 3. The resulting *Escherichia coli* was inoculated in 2 mL of a Plusgrow II medium (4% Plusgrow II (manufactured by Nacalai Tesque, Inc.) and 0.005% carbenicillin disodium). The resulting mixture was cultured with shaking at 37°C for 6 hours to obtain a preculture liquid. Into 1.4 mL of a Plusgrow II medium (4%Plusgrow II and 0.005% carbenicillin disodium) in a test tube, 100 µL of the preculture liquid was inoculated. The resulting mixture was cultured with shaking at 37°C for 16 hours.

Then, for the purpose of expression induction, 1.5 mL of a Plusgrow II medium (4% Plusgrow II and 0.005% carbenicillin disodium) and 30 µL of an arabinose solution (10% arabinose) were added, in which the medium was adjusted in advance to pH 7.0, pH 7.5, pH 8.0, and pH 9.0 using a 1 M NaOH solution. The resulting solution was cultured with shaking for 6 hours. Then, bacterial bodies were collected through centrifugation. The 6-hour shaking culture was a shaking culture performed at 37°C for 6 hours (Method A) or a shaking culture performed at 30°C for 4 hours and then at 37°C for 2 hours (Method B).

DNA was extracted using the same method as in "(4) DNA extraction" in Example 2.

### (2) Evaluation of total DNA yield

Furthermore, the total DNA yield was evaluated using the same method as in "(5) Evaluation of total DNA yield" in Example 2. The results are shown in Table 3 below.

**[Table 3]**

| Table 3: Measurement results of total DNA yield | | | |
|---|---|---|---|
| Vector for producing linear covalently closed DNA | Method A^{*1} /Method B^{*2} | pH of Plusgrow II medium^{*3} | Total DNA yield (µg) from 3.0 mL of culture liquid |
| Control vector | Method A | 7.0 | 37.5 |
| | | 8.0 | 39.6 |
| | Method B | 7.0 | 27.0 |
| | | 7.5 | 30.3 |
| | | 8.0 | 29.3 |
| | | 9.0 | 27.6 |
| Separate expression vector (T6) | Method A | 7.0 | 29.9 |
| | | 8.0 | 28.7 |
| | Method B | 7.0 | 28.5 |
| | | 7.5 | 30.2 |
| | | 8.0 | 27.4 |
| | | 9.0 | 30.6 |
| Fusion expression vector (T6) | Method A | 7.0 | 20.2 |
| | | 8.0 | 21.0 |
| | Method B | 7.0 | 21.2 |
| | | 7.5 | 19.2 |
| | | 8.0 | 20.6 |
| | | 9.0 | 20.7 |

| | | | |
|---|---|---|---|
| Method A^{*1}: shaking culture at 37°C for 6 hours Method B^{*2}: shaking culture at 30°C for 4 hours and then shaking culture at 37°C for 2 hours pH of Plusgrow II medium^{*3}: adjusted in advance using a 1 M NaOH solution | | | |

### (3) Evaluation of linear covalently closed DNA

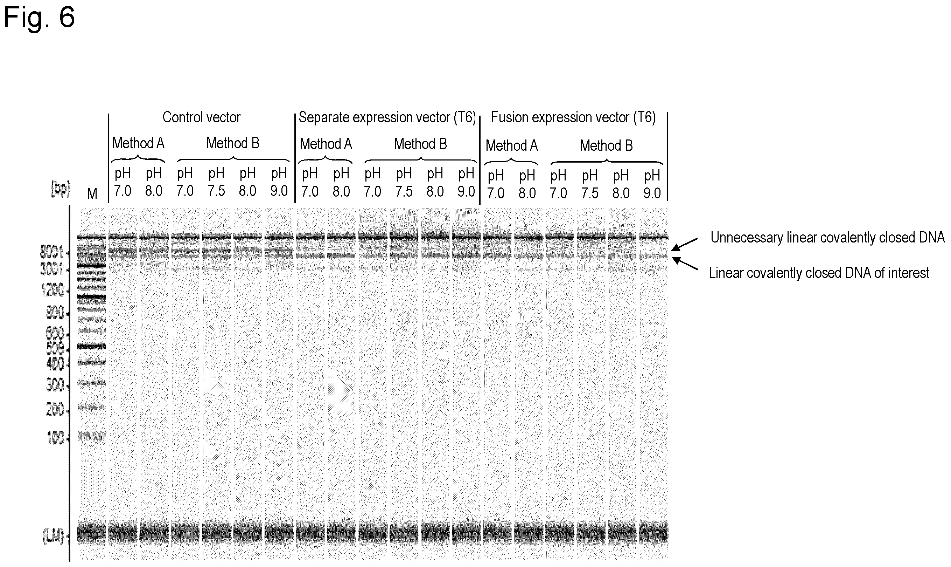

The results obtained by analyzing a linear covalently closed DNA using the same method as in "(6) Evaluation of linear covalently closed DNA" in Example 2 are shown in Figure 6. Furthermore, the results obtained by calculating the residual ratio of an unnecessary linear covalently closed DNA are shown in Table 4 below.

**[Table 4]**

| Table 4: Residual ratio of unnecessary linear covalently closed DNA | | | |
|---|---|---|---|
| Vector for producing linear covalently closed DNA | Method A^{*1} /Method B^{*2} | pH of Plusgrow II medium^{*3} | Residual ratio (%) of unnecessary linear covalently closed DNA |
| Control vector | Method A | 7.0 | 92.6 |
| | | 8.0 | 90.6 |
| | Method B | 7.0 | 109.4 |
| | | 7.5 | 87.1 |
| | | 8.0 | 101.2 |
| | | 9.0 | 93.3 |
| Separate expression vector (T6) | Method A | 7.0 | 24.8 |
| | | 8.0 | 21.9 |
| | Method B | 7.0 | 57.8 |
| | | 7.5 | 48.0 |
| | | 8.0 | 46.6 |
| | | 9.0 | 35.0 |
| Fusion expression vector (T6) | Method A | 7.0 | 36.6 |
| | | 8.0 | 26.6 |
| | Method B | 7.0 | 45.1 |
| | | 7.5 | 37.2 |
| | | 8.0 | 34.7 |
| | | 9.0 | 9.2 |

| | | | |
|---|---|---|---|
| Method A^{*1}: shaking culture at 37°C for 6 hours Method B^{*2}: shaking culture at 30°C for 4 hours and then shaking culture at 37°C for 2 hours pH of Plusgrow II medium^{*3}: adjusted in advance using a 1 M NaOH solution | | | |

In the results in Figure 6 and Table 4, the residual ratio of an unnecessary linear covalently closed DNA decreased significantly in the *Escherichia coli* body having a separate expression vector or fusion expression vector transduced thereto, compared with the *Escherichia coli* body having a control vector transduced thereto. These results suggest that, in both the separate expression vector and the fusion expression vector, the homing endonuclease recognition sequence was cleaved by the I-SceI homing nuclease, and degradation by the endogenous exonuclease in the *Escherichia coli* body was caused from the cleaved end.

On the basis of the results in Figure 6 and Table 4, the culture temperature in the expression-induction step is not particularly limited, as long as the protelomerase and the homing endonuclease function in a cell in the range. However, under the condition (Method A) of shaking culture at 37°C for 6 hours after the addition of an arabinose solution, the residual ratio of the unnecessary linear covalently closed DNA from the separate expression vector was lower than from the fusion expression vector. On the other hand, under the condition (Method B) of shaking culture at 37°C for 2 hours after shaking culture at 30°C for 4 hours after the addition of an arabinose solution, the residual ratio of the unnecessary linear covalently closed DNA from the fusion expression vector was lower than from the separate expression vector.

These results suggest that adjusting the temperature to 30°C after the addition of an arabinose solution results in increasing the efficiency of production of a linear covalently closed DNA owing to the TelN protelomerase activity, and then shifting the temperature to 37°C results in increasing the I-SceI homing endonuclease activity, thus increasing the efficiency of degradation of an unnecessary linear covalently closed DNA, because the optimal temperature for the enzymic activity of TelN protelomerase was 30°C, and the optimal temperature for the enzymic activity of I-SceI homing endonuclease was 37°C.

Similarly, on the basis of the results in Figure 6 and Table 4, the pH in the expression-induction step is not particularly limited, as long as the protelomerase and the homing endonuclease function in a cell in the range. However, in Method A, both the separate expression vector and the fusion expression vector caused the residual ratio of an unnecessary linear covalently closed DNA to be lower at pH 8.0 than at pH 7.0 as the pH of the culture medium added in the expression-induction step. Furthermore, in Method B, both the separate expression vector and the fusion expression vector caused the residual ratio of an unnecessary linear covalently closed DNA to be lower as the pH of the culture medium added in the expression-induction step increased from 7.0 to 9.0.

These results suggest that increasing, from 7.0 to 9.0, the pH of the culture medium added in the expression-induction step results in increasing the efficiency of production of a linear covalently closed DNA owing to the TelN protelomerase activity, and furthermore, results in increasing the I-SceI homing endonuclease activity, thus increasing the efficiency of degradation of an unnecessary linear covalently closed DNA, because the optimal pH for the enzymic activity of the TelN protelomerase was 8.8, and the optimal temperature for the enzymic activity of the I-SceI homing endonuclease was pH 7.9.

### <Example 5: Study on fusion polypeptide>

### (Purpose)

The purpose is to compare the efficiency of production of a linear covalently closed DNA through the production of a fusion polypeptide containing TelN protelomerase at the N-terminal side and I-SceI homing endonuclease at the C-terminal side (the fusion polypeptide is hereinafter referred to as a "PH type fusion polypeptide") and a fusion polypeptide containing I-SceI homing endonuclease at the N-terminal side and TelN protelomerase at the C-terminal side (the fusion polypeptide is hereinafter referred to as an "HP type fusion polypeptide").

### (Method and results)

### (1) Production of PH type and HP type fusion expression vectors

The "fusion expression vector (T0)" produced in Example 2 was used as a vector for producing a linear covalently closed DNA comprising a gene encoding a PH type fusion polypeptide comprising TelN protelomerase, a linker sequence (an amino acid sequence encoded by the base sequence of SEQ ID NO: 34), and I-SceI homing endonuclease in this order from the N-terminal side (hereinafter referred to as a "PH type fusion expression vector"). The PH type fusion expression vector was designed so that a PH type fusion polypeptide could be expressed under the control of an arabinose induction promoter.

A vector for producing a linear covalently closed DNA comprising a gene encoding an HP type fusion polypeptide comprising I-SceI homing endonuclease, a linker sequence (amino acid sequence encoded by the base sequence of SEQ ID NO: 34), and TelN protelomerase in this order from the N-terminal side (hereinafter referred to as a "HP type fusion expression vector") was produced by interchanging the region encoding the TelN protelomerase with the region encoding the I-SceI homing endonuclease in the above-described PH type fusion expression vector. The HP type fusion expression vector was designed so that an HP type fusion polypeptide could be expressed under the control of an arabinose induction promoter.

### (2) Evaluation of amount of production of linear covalently closed DNA

Using the PH type fusion expression vector or the HP type fusion expression vector, and using the same method as in Example 3, the transformation, culture, expression induction, DNA extraction, and evaluation of total DNA yield, and evaluation of a linear covalently closed DNA was carried out. However, in this Example, a shaking culture for 6 hours in the presence of arabinose was performed at 30°C using 200 mL of the culture liquid.

Table 5 below shows the results obtained by calculating the total DNA yield obtained from 200 mL of the culture liquid based on a measurement made using Nanodrop.

**[Table 5]**

| Table 5: Measurement results of total DNA yield | |
|---|---|
| Vector for producing linear covalently closed DNA | Total DNA yield (mg) from 200 mL of culture liquid |
| PH type fusion expression vector | 3.07 |
| HP type fusion expression vector | 2.06 |
| Control vector | 3.15 |

Next, the results obtained by evaluating the residual ratio of an unnecessary linear covalently closed DNA using electrophoresis are shown in Table 6 below.

**[Table 6]**

| Table 6: Residual ratio of unnecessary linear covalently closed DNA | |
|---|---|
| Vector for producing linear covalently closed DNA | Residual ratio (%) of unnecessary linear covalently closed DNA |
| PH type fusion expression vector | 32.32 |
| HP type fusion expression vector | 40.75 |
| Control vector | 85.63 |

The results shown in Tables 5 to 6 have revealed that the total DNA yield was higher, and the residual ratio of an unnecessary linear covalently closed DNA was lower, from the PH type fusion expression vector than from the HP type fusion expression vector.

These results show that, as expected from the structure prediction in Example 2, placing the TelN protelomerase at the N-terminal side of the I-SceI homing endonuclease enables both the protelomerase activity and homing endonuclease activity of the fusion polypeptide to function efficiently.

### <Comparative Example 1: Construction of two vectors for producing linear covalently closed DNA>

### (Purpose)

The purpose is to produce a control vector (T6) and an I-SceI vector as two vectors for producing a linear covalently closed DNA.

### (Method and results)

### (1) Production of nucleic acid fragment

A nucleic acid (SEQ ID NO: 35) encoding aminoglycoside 3'-phosphotransferase which confers kanamycin resistance was produced, through total synthesis, as a nucleic acid fragment to be used to construct a vector.

### (2) Production of control vector (T6)

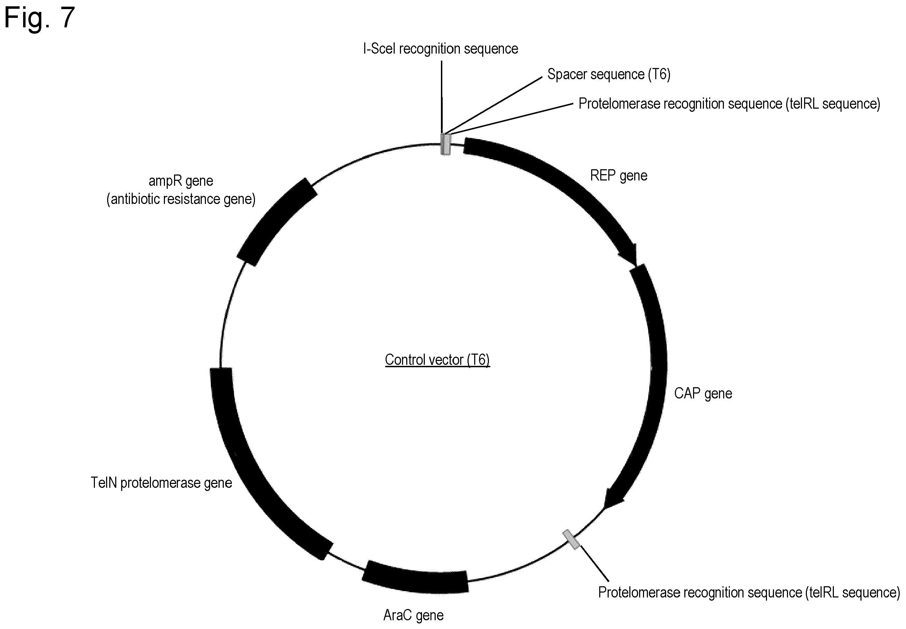

A vector for expressing TelN protelomerase under the control of an arabinose induction promoter (hereinafter referred to as a "control vector (T6)") was produced (Figure 7), in which the vector comprised T6 as a spacer sequence between the telRL sequence and the I-SceI recognition sequence.

The method of producing a control vector (T6) was as follows. First, a nucleic acid fragment was produced through a PCR using, as a template, the separate expression vector (T6) produced in Example 1, and using a primer 9 (forward, SEQ ID NO: 36) and a primer 10 (reverse, SEQ ID NO: 37). Next, utilizing the overlapping region at an end of the resulting nucleic acid fragment, a control vector (T6) was produced through circularization by a Gibson Assembly system of New England Biolabs (Figure 7). The control vector (T6) is a vector resulting from excluding, from the separate expression vector (T6), the I-SceI homing endonuclease gene designed to be expressed under the control of arabinose induction promoter. In the control vector (T6), the Rep gene and the Cap gene were placed between a pair of protelomerase recognition sequences (telRL sequences) (Figure 7).

### (3) Production of I-SceI vector

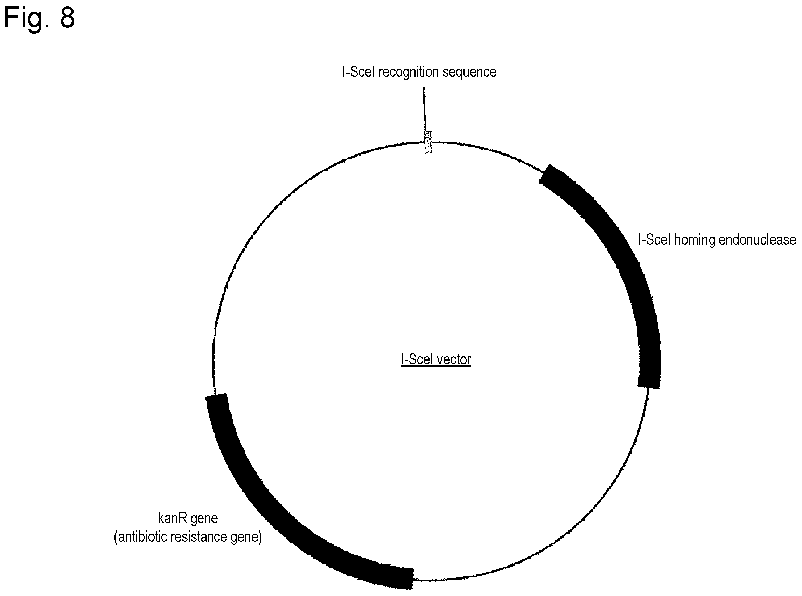

A vector for expressing an I-SceI homing endonuclease under the control of an arabinose induction promoter (hereinafter referred to as an "I-SceI vector") was produced (Figure 8), in which the vector comprised a nucleic acid (SEQ ID NO: 35) encoding aminoglycoside 3'-phosphotransferase which confers kanamycin resistance.

The method of producing an I-SceI vector was as follows. First, a nucleic acid encoding β lactamase which confers ampicillin resistance and was comprised in the separate expression vector (T6) produced in Example 1 was substituted with a nucleic acid (SEQ ID NO: 35) encoding aminoglycoside 3'-phosphotransferase which confers kanamycin resistance. A nucleic acid fragment was produced through a PCR using, as a template, the vector produced through the substitution, and using the primer 7 (forward, SEQ ID NO: 20) and a primer 11 (reverse, SEQ ID NO: 38). Next, utilizing the overlapping region at an end of the resulting nucleic acid fragment, an I-SceI vector was produced through circularization by a Gibson Assembly system of New England Biolabs (Figure 8). The I-SceI vector did not comprise the protelomerase recognition sequence (telRL sequence), but comprised the I-SceI recognition sequence (Figure 8).

### <Comparative Examples 2: Production of linear covalently closed DNA>

### (Purpose)

The purpose is to transform *Escherichia coli* with two vectors produced in Comparative Example 1, and thereby producing a linear covalently closed DNA. The purpose is also to evaluate the amount of production of a linear covalently closed DNA.

### (Method and results)

### (1) Transformation

*Escherichia coli* was transformed with the control vector (T6) and the I-SceI vector which were produced in Comparative Example 1. In addition, *Escherichia coli* transformed with the control vector (T6) alone was produced as a control group.

A competent cell solution of an *Escherichia coli DH10B* strain, in an amount of 25 µL, and a solution comprising each vector were mixed and left to stand on ice for 30 minutes. After standing for 30 minutes, the resulting solution was heat-treated at 42°C for 45 seconds, and left to stand on ice for 2 minutes. Then, 225 µL of an SOC culture medium was added. In a case where the control vector (T6) alone was transduced, the *Escherichia coli* was applied to an LB agar culture medium (1% tryptone, 0.5% dry yeast extract, 1% sodium chloride, and 0.005% carbenicillin disodium (manufactured by Nacalai Tesque, Inc.)). In a case where the control vector (T6) and the I-SceI vector were transduced, the *Escherichia coli* was applied to an LB agar culture medium (1% tryptone, 0.5% dry yeast extract, 1% sodium chloride, 0.005% carbenicillin disodium (manufactured by Nacalai Tesque, Inc.) and 0.005% kanamycin sulfate (manufactured by Fujifilm Wako Pure Chemical Corporation)). A strain which grew in a stationary culture performed at 37°C for 1 day was selected, and *Escherichia coli* having a vector transduced thereto was obtained.

### (2) Culture

The *Escherichia coli* obtained in Example 3 having the separate expression vector (T6) transduced thereto was inoculated into 2 mL of a Plusgrow II medium (4% Plusgrow II (manufactured by Nacalai Tesque, Inc.) and 0.005% carbenicillin disodium). The resulting mixture was cultured with shaking at 37°C for 6 hours to obtain a preculture liquid. Into 100 mL of a Plusgrow II medium (4%Plusgrow II and 0.005% carbenicillin disodium) in a flask, 200 µL of the preculture liquid was inoculated. The resulting mixture was cultured with shaking at 37°C for 16 hours.

The *Escherichia coli* obtained as described above having the control vector (T6) alone transduced thereto was inoculated into 2 mL of a Plusgrow II medium (4% Plusgrow II and 0.005% carbenicillin disodium). The resulting mixture was cultured with shaking at 37°C for 6 hours to obtain a preculture liquid. Into 100 mL of a Plusgrow II medium (4%Plusgrow II and 0.005% carbenicillin disodium) in a flask, 200 µL of the preculture liquid was inoculated. The resulting mixture was cultured with shaking at 37°C for 16 hours.

The *Escherichia coli* obtained as described above having the control vector (T6) and the I-SceI vector transduced thereto was inoculated into 2 mL of a Plusgrow II medium (4% Plusgrow II, 0.005% carbenicillin disodium, and 0.005% kanamycin sulfate). The resulting mixture was cultured with shaking at 37°C for 6 hours to obtain a preculture liquid. Into 100 mL of a Plusgrow II medium (4%Plusgrow II, 0.005% carbenicillin disodium, and 0.005% kanamycin sulfate) in a flask, 200 µL of the preculture liquid was inoculated. The resulting mixture was cultured with shaking at 37°C for 16 hours.

### (3) Expression induction

To the culture liquid obtained from the *Escherichia coli* having the separate expression vector (T6) transduced thereto, 100 mL of a Plusgrow II medium (4% Plusgrow II and 0.005% carbenicillin disodium) having pH 7.0 and 2 mL of an arabinose solution (10% arabinose) were added. The resulting mixture was cultured with shaking at 30°C or 37°C for 6 hours. Then, bacterial bodies were collected through centrifugation.

To the culture liquid obtained from the *Escherichia coli* having the control vector (T6) alone transduced thereto, 100 mL of a Plusgrow II medium (4% Plusgrow II and 0.005% carbenicillin disodium) having pH 7.0 and 2 mL of an arabinose solution (10% arabinose) were added. The resulting mixture was cultured with shaking at 30°C or 37°C for 6 hours. Then, bacterial bodies were collected through centrifugation.

To the culture liquid obtained from the *Escherichia coli* having the control vector (T6) and the I-SceI vector transduced thereto, 100 mL of a Plusgrow II medium (4% Plusgrow II, 0.005% carbenicillin disodium, and 0.005% kanamycin sulfate) having pH 7.0 and 2 mL of an arabinose solution (10% arabinose) were added. The resulting mixture was cultured with shaking at 30°C or 37°C for 6 hours. Then, bacterial bodies were collected through centrifugation.

### (4) DNA extraction

From 1 mL of the culture liquid collected, DNA was extracted using a QIA prep Spin Miniprep Kit (manufactured by QIAGEN N.V.).

### (5) Evaluation of total DNA yield

Table 7 below shows the results obtained by calculating the total DNA yield obtained from 200 mL of the culture liquid based on a measurement made using Nanodrop (manufactured by Thermo Fisher Scientific Inc.).

**[Table 7]**

| Table 7: Measurement results of total DNA yield | | |
|---|---|---|
| Vector for producing linear covalently closed DNA | Total DNA yield (mg) from 200 mL of culture liquid | |
| | Expression-induction culture temperature: 30°C | Expression-induction culture temperature: 37°C |
| Separate expression vector (T6) | 2.64 | 2.46 |
| Control vector (T6) | 4.51 | 3.92 |
| Control vector (T6) and I-Scel vector | 2.67 | 2.31 |

### (6) Evaluation of linear covalently closed DNA

A linear covalently closed DNA was evaluated using electrophoresis. Specifically, the DNA solution obtained in (4) above was adjusted to 2 to 10 ng/µL, and analyzed using a microchip electrophoresis system MultiNA for analyzing DNA/RNA (manufactured by Shimadzu Corporation).

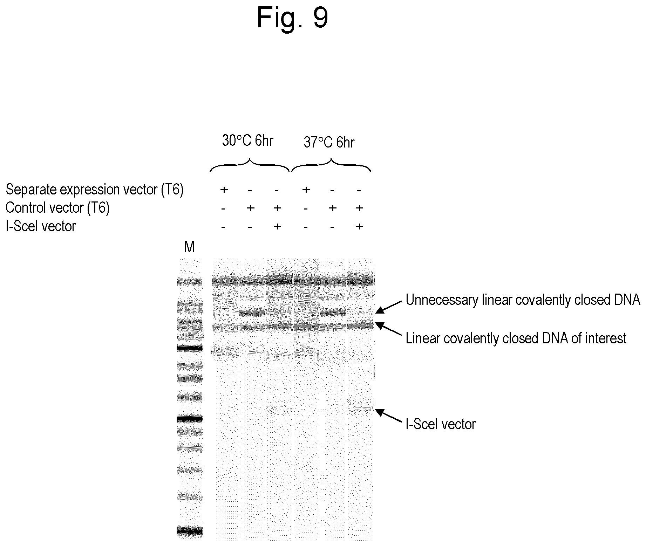

Figure 9 shows the electrophoresis results obtained using MultiNA. In Figure 9, a linear covalently closed DNA comprising a nucleic acid encoding a packaging protein is shown as a "linear covalently closed DNA of interest", and a linear covalently closed DNA derived from a vector sequence other than the nucleic acid encoding a packaging protein is shown as an "unnecessary linear covalently closed DNA". In the diagram, the left-side image shows the shaking culture performed at an expression-induction culture temperature of 30°C for 6 hours, and the right-side image shows a sample cultured with shaking at an expression-induction culture temperature of 37°C for 6 hours.

The results shown in Figure 9 have revealed that using *Escherichia coli* having the control vector (T6) and the I-SceI vector transduced thereto caused part of the I-SceI vector to remain, thus causing DNA other than a linear covalently closed DNA of interest to increase.

All publications, patents, and patent applications cited herein are incorporated herein by reference in their entirety.

## Claims

1. A double-stranded circular DNA vector, comprising
a protelomerase gene sequence encoding a protelomerase or an active fragment thereof,
an endonuclease gene sequence encoding an endonuclease or an active fragment thereof,
a pair of protelomerase recognition sequences which are recognized by said protelomerase or an active fragment thereof for cleaving said vector,
at least one endonuclease recognition sequence which is recognized by said endonuclease or an active fragment thereof for cleaving said vector, and
a nucleic acid sequence of interest,
wherein said protelomerase gene sequence, said endonuclease gene sequence, and said endonuclease recognition sequence are placed in the same region between said pair of protelomerase recognition sequences, and said nucleic acid sequence of interest is placed in another region between said pair of protelomerase recognition sequences in said double-stranded circular DNA vector, and
wherein said protelomerase gene sequence and said endonuclease gene sequence are placed under the control of a promoter which is capable of regulating the expression.

2. The double-stranded circular DNA vector of claim 1, wherein said protelomerase gene sequence and said endonuclease gene sequence encode a fusion polypeptide comprising said protelomerase or an active fragment thereof and said endonuclease or an active fragment thereof.

3. The double-stranded circular DNA vector of claim 2, wherein said protelomerase or an active fragment thereof is placed at an N-terminal side of said endonuclease or an active fragment thereof in said fusion polypeptide.

4. The double-stranded circular DNA vector of claim 2, wherein said fusion polypeptide further comprises a linker sequence between said protelomerase or an active fragment thereof and said endonuclease or an active fragment thereof.

5. The double-stranded circular DNA vector of claim 1 or 2, further comprising a spacer sequence which is placed between said endonuclease recognition sequence and said protelomerase recognition sequence.

6. The double-stranded circular DNA vector of claim 5, wherein said spacer sequence is 200 bases in length or less.

7. The double-stranded circular DNA vector of claim 1 or 2, wherein said endonuclease recognition sequence is placed adjacent to one of said protelomerase recognition sequences.

8. The double-stranded circular DNA vector of claim 1, which is a plasmid.

9. The double-stranded circular DNA vector of claim 1, wherein said protelomerase is TelN protelomerase, TelA protelomerase, or TelK protelomerase.

10. The double-stranded circular DNA vector of claim 1, wherein said endonuclease is a homing endonuclease.

11. The double-stranded circular DNA vector of claim 10, wherein said homing endonuclease is I-SceI homing endonuclease, I-CeuI homing endonuclease, or I-CreI homing endonuclease.

12. The double-stranded circular DNA vector of claim 1, wherein said nucleic acid sequence of interest comprises a gene sequence of interest encoding a protein of interest or a fragment thereof.

13. A transformed cell comprising the double-stranded circular DNA vector of claim 1.

14. The transformed cell of claim 13, wherein the cell is a bacterial cell or a eukaryotic cell.

15. The transformed cell of claim 14, wherein the cell is *Escherichia coli* or *Bacillus subtilis.*

16. The transformed cell of claim 13, which expresses an exonuclease.

17. A method of producing a linear covalently closed DNA, comprising
a culture step of culturing the transformed cell of claim 13,
an expression-induction step of inducing the expression of said protelomerase or an active fragment thereof and said endonuclease or an active fragment thereof in the transformed cell after said culture step, and
a DNA extraction step of extracting DNA from the transformed cell after said expression-induction step.

18. The method of claim 17, further comprising a separation step of separating the linear covalently closed DNA after said DNA extraction step.

19. The method of claim 17, further comprising a transduction step of transducing the double-stranded circular DNA vector of claim 1 to a host cell before said culture step for producing the transformed cell.

20. The method of claim 17, wherein said transformed cell expresses an exonuclease.

21. A fusion polypeptide comprising a protelomerase or an active fragment thereof at an N-terminal side and an endonuclease or an active fragment thereof at a C-terminal side, or a nucleic acid encoding the same.
